# EUROPÄISCHE PATENTSCHRIFT

(11) **EP 3 569 282 B1**
(45) Veröffentlichungstag und Bekanntmachung des Hinweises auf die Patenterteilung: **17.04.2024**
(21) Anmeldenummer: 19159970.3
(22) Anmeldetag: 28.02.2019
(51) Int. Cl.: A61M 39/26, A61M 39/04

(54) **VENTIL MIT VORGEFERTIGTER KOMPRIMIERTER DILATIERBARER ÖFFNUNG**
VALVE WITH PREFABRICATED COMPRESSED DILATABLE OPENING
SOUPAPE À OUVERTURE DILATABLE COMPRESSÉE PRÉFABRIQUÉE

(30) Priorität: 16.05.2018 DE 102018111815
(43) Veröffentlichungstag der Anmeldung: 20.11.2019
(73) Patentinhaber: CODAN Medizinische Geräte GmbH, 23738 Lensahn (DE)
(72) Erfinder: Behl, Christoph, 23701 Eutin (DE)
(74) Vertreter: Raffay & Fleck

(56) Entgegenhaltungen:
- US-A- 5 676 346
- US-A- 5 814 024
- US-B1- 6 428 520
- US-B2- 7 306 199

## Beschreibung

Die Erfindung betrifft ein Ventil, welches einen Fluidaustausch mit einer nadelfreien Spritze ermöglicht.

Ventile, die einen Zugang zum Beispiel für eine nadelfreie Spritze ermöglichen, um so Flüssigkeiten zwischen Spritze und einer Anordnung, an der das Ventil angebracht ist, ermöglichen, sind im Stand der Technik zahlreich bekannt.

So gibt es zum einen Ventile die eine Membran aufweisen, in die ein Schlitz eingebracht ist. Schlitze weisen dabei in einem Ruhezustand, in dem die Membran nicht komprimiert oder verformt ist keinen geöffneten Fluidkanal auf, weshalb ein Abdichten des Schlitz gegen Flüssigkeitsdurchtritt bei geringem Druck durch eine leichte Komprimierung der Membran relativ einfach zu bewerkstelligen ist und ein dichter Verschluss des Ventils somit gewährleistet werden kann. Eine solches Ventil mit einer Membran, in der ein Schlitz eingebracht ist, ist beispielsweise in der US 6,428,520 B1 oder in der US 2016/0354594 A1 gegeben, wobei für letzte angegeben ist, dass hier die Membran leicht komprimiert wird, so dass der Schlitz einem Druck von bis zu etwa 200 kPa (30 psi) standhält. Allerdings stellt ein solches Ventil lediglich mit einem Schlitz in der Membran vor allem das Problem dar, dass sich solche Schlitze beim Einführen der Spritze nicht allzu weit öffnen lassen. Ein weiterer Nachteil besteht darin, dass bei Schlitzanordnung die Gefahr besteht, dass bisweilen der Schlitz verstopft. Auch ist für das Einbringen des Schlitzes ein weiterer Arbeitsschritt erforderlich.

Aufgrund dessen sind andere Ausgestaltungen eines Ventils bekannt, die in ihrer Membran eine vorgefertigte Öffnung aufweisen. Eine solche Öffnung wird bei der Produktion in die Membran mit gefertigt. Somit weist eine solche Membran im Gegensatz zu Membranen mit Schlitz nach der Produktion einen geöffneten Fluidkanal auf. Eine solche Öffnung vermindert einige Nachteile, die eine Membran mit Schlitz aufweist, allerdings entsteht das Problem, dass das Ventil, wenn es nicht z. B. mit der nadelfreien Spritze verbunden und durch diese geöffnet wird, so verschlossen sein muss, dass keine Flüssigkeit ein bzw. keine Flüssigkeit austreten kann. Daher muss zum einen die vorgefertigte Öffnung klein sein und zum anderen diese dicht verschlossen werden. Dazu sind aus dem Stand der Technik mehrere Alternativen bekannt.

Die US 5,814,024 offenbart eine Membran die so ausgeformt ist, dass sie eine konkave äußere Oberfläche aufweist und die Öffnung in der Membran aufgrund dieser Geometrie der Oberfläche schließt. Diese Druckschrift offenbart ein Ventil gemäß dem Oberbegriff des Anspruchs 1. Die US 9,138,572 offenbart eine Membran, die von einer steiferen Ummantelung umgeben ist, die sich allerdings beim Öffnen des Ventils mit der Membran im Ventil bewegt, und dabei eine Öffnung in der Membran öffnet und schließt. Die US 5,676,346 offenbart eine Membran, an welcher an der Unterseite der Öffnung der Membran zusätzlich Zuspitzungen angeordnet sind, die so ausgewählt sind, dass diese das Schließen der Öffnung aufgrund des Innendrucks, der durch die innerhalb der Membran befindlichen Flüssigkeit erzeugt wird, bewirkt. All diesen Ventilen ist gemeinsam, dass der Abschnitt der Membran, der die Öffnung aufweist, in einem endständigen Abschnitt des Ventils angeordnet ist. Dabei wird zumindest in der US 5,676,346 die Öffnung der Membran nicht nur aufgrund der Zuspitzungen an ihrer Unterseite geschlossen, sondern auch durch eine engere Fassung des Ventils an diesem endständigen Abschnitt komprimiert. Eine solche Komprimierung der Öffnung ist auch in der US 7,306,199 B2 offenbart, wobei die Öffnung beim Verbringen des endständigen Abschnitts in einen breiteren Abschnitts des Ventils in ihren ursprünglichen, nicht-kompressierten Zustand zurückfällt.

Beim Einführen der nadelfreien Spritze wird dieser obere Abschnitt der Membran aus diesem oberen Bereich in einen breiteren Bereich des Ventils verschoben, wobei durch den breiteren Bereich die natürliche Ausdehnung der Öffnung der Membran wiederhergestellt wird.

Ein Problem der vorbekannten Lösungen liegt darin, dass bei einer möglichst gro-ßen Öffnung der Membran diese Öffnung zum einen sicher geschlossen gehalten werden soll und gleichzeitig allerdings ein effektiver und häufig wiederholbarer Fluidaustausch ermöglicht werden soll, wenn eine nadelfreie Spritze in das dem Ventil eingeführt wird.

Entsprechend besteht die Aufgabe der Erfindung darin, Nachteile der derzeitigen Ventile zu adressieren und minimieren bzw. zu beseitigen.

Die Aufgabe wird erfindungsgemäß gelöst durch ein Ventil mit den Merkmalen des Anspruchs 1. Vorteilhafte Weiterbildungen eines solchen Ventils sind in den abhängigen Ansprüchen 2 - 11 angegeben. Die Aufgabe wird ebenso gelöst durch ein Verfahren zur Erstellung eines Ventils mit den Merkmalen des Verfahrensanspruchs 12 und vorteilhafte Weiterbildungen eines solchen Verfahrens mit den Ansprüchen 13 und 14.

Dabei weist das Ventil erfindungsgemäß einen Ventilkörper, insbesondere mit einer endständigen Fluidöffnung, insbesondere am Ende eines endständigen Fluidkanales, auf. Zudem weist der Ventilkörper eine Ventilöffnung auf, die sich endständig an einem Öffnungskanal des Ventilkörpers befindet, insbesondere der Fluidöffnung gegenüberliegend. Dabei sind die Ventilöffnung und der Öffnungskanal zur Aufnahme eines Tubus durch Einführen des Tubus durch die Ventilöffnung hindurch in den Öffnungskanal eingerichtet. Vorteilhafterweise erfolgt mithilfe des Tubus ein Fluidaustausch, insbesondere ein Hinzufügen eines Fluids mithilfe des Tubus in das Ventil und/oder eine Entnahme eines Fluids mithilfe des Tubus aus dem Ventil. Vorteilhafterweise ist der Tubus eine nadelfreie Spritze oder Teil einer nadelfreien Spritze. Insbesondere ist der Öffnungskanal konisch ausgebildet, wobei ein weiterer oder breiterer Querschnitt des konisch ausgebildeten Öffnungskanals an der Ventilöffnung angrenzt und insbesondere ein kleinerer Querschnitt weiter im Inneren des Ventilkörpers angeordnet ist. Vorteilhafterweise wird die Aufnahme eines Tubus durch die konische Form und/oder konische Verjüngung des Öffnungskanal begrenzt. Vorteilhafterweise lässt die Ventilöffnung und/oder der Öffnungskanal einen Fluidaustausch und/oder Fluidtransport zwischen Ventil und Tubus zu. Vorteilhafterweise handelt es sich bei dem Fluid um eine Flüssigkeit.

Erfindungsgemäß weist das Ventil einen elastisch deformierbaren Verschlusskörper innerhalb des Ventilkörpers auf, wobei der Verschlusskörper eine endständige, insbesondere ebene, Oberfläche und in einem ersten Abschnitt einen ersten Fluidkanal aufweist. Dieser Verschlusskörper ist vorteilhafterweise so ausgebildet und angeordnet, dass er in einem Ruhezustand des Ventils, in dem das Ventil durch den Verschlusskörper verschlossen ist, an der Ventilöffnung angrenzt und sich auch entlang des Öffnungskanals erstreckt und sich insbesondere innerhalb des Ventilkörpers von der Ventilöffnung ausgehend bis hin zu einem dem Öffnungskanal gegenüber weiter geöffneten und/oder breiteren Kanal des Ventilkörpers erstreckt.

Erfindungsgemäß ist der Verschlusskörper so ausgebildet, dass er in einem undeformierten Zustand des Verschlusskörpers, in dem der Verschlusskörper insbesondere nicht komprimiert ist und der insbesondere nicht seinem Zustand im Ruhezustand des Ventils entspricht, in einem zweiten und endständigen Abschnitt einen zweiten Fluidkanal aufweist, der mit dem ersten Fluidkanal verbunden ist, wobei vorteilhafterweise der zweite Fluidkanal an einer endständigen Oberfläche des Verschlusskörpers mündet, die insbesondere der endständigen Fluidöffnung und/oder dem endständigen Fluidkanale und/oder dem ersten Abschnitt und/oder dem ersten Fluidkanal abgewandt und/oder gegenüberliegend angeordnet ist. Dabei ist der undeformierte Zustand des Verschlusskörpers vorteilhafterweise dadurch gekennzeichnet, dass in dem undeformierten Zustand des Verschlusskörpers keine Kraft, insbesondere keine Kompressionskräfte, auf den Verschlusskörper einwirken. Ein solcher Zustand muss bei Anordnung im Ventilkörper nicht vorliegen können.

Erfindungsgemäß ist in einem Ruhezustand des Ventils, in dem das Ventil durch den Verschlusskörper verschlossen ist, der Verschlusskörper mit seinem zweiten Abschnitt in dem Öffnungskanal des Ventilkörpers so angeordnet, so dass der zweite Abschnitt so deformiert ist, dass der zweite Fluidkanal verschlossen ist. Dabei wirkt vorteilhafterweise in dem Ruhezustand des Ventils auf den Verschlusskörper eine Kraft, insbesondere eine komprimierende Kraft, ein, insbesondere ausgeübt durch den Öffnungskanal.

Erfindungsgemäß ist das Ventil so eingerichtet, dass durch Einführen des Tubus durch die Ventilöffnung in den Öffnungskanal unter Ausübung einer Kraft mit dem Tubus auf die endständige Oberfläche das Ventil in einen offenen, auch geöffneten, Zustand verbracht wird, wobei der zweite Abschnitt aus dem Öffnungskanal in einen im Querschnitt größeren und/oder breiteren Kanal des Ventilkörpers überführt wird, und wobei in dem offenen Zustand der zweite Fluidkanal eine Verbindung zwischen dem ersten Fluidkanal, insbesondere auch der Fluidöffnung, und dem Tubus ermöglicht. Dabei ist vorteilhafterweise der größere und/oder breitere Kanal des Ventilkörper dadurch gekennzeichnet, dass er und/oder seine Weite im Querschnitt größer bzw. breiter ist als der Öffnungskanal, insbesondere weist der größere Kanal einen größeren Innenquerschnitt und/oder eine größere freie Innenquerschnittsfläche auf als der Innenquerschnitt und/oder eine freie Innenquerschnittsfläche des Öffnungskanals senkrecht zur Längserstreckung des Ventils und/oder Verschlusskörpers und/oder parallel zur Ebene der Ventilöffnung und/oder endständigen Oberfläche auf, insbesondere ist der größere Kanal breiter in seinem inneren freien Querschnitt und/oder Innendurchmesser als der Öffnungskanal in seinem inneren freien Querschnitt und/oder Innendurchmesser.

Erfindungsgemäß ist das Ventil so eingerichtet, dass ausgehend vom offenen Zustand beim Entfernen des Tubus aus dem Öffnungskanal, und insbesondere der Ventilöffnung, der zweite Abschnitt so in den Öffnungskanal zurückdrängt und/oder zurück gedrückt wird, insbesondere durch den Ventilkörper, dass der Ruhezustand hergestellt wird.

Erfindungsgemäß und wesentlich ist das Ventil dabei so eingerichtet, dass unter Einführung des Tubus durch die Ventilöffnung in den Öffnungskanal das Ventil aus seiner Ruhelage in den offenen Zustand gebracht wird und dadurch der Verschlusskörper so deformiert wird, dass der zweite Fluidkanal über den undeformierten Zustand des Verschlusskörpers hinaus dilatiert wird. Dabei ist der Verschlusskörper des Ventils erfindungsgemäß so ausgebildet, dass diese Dilatation unmittelbar und allein durch Krafteinwirkung auf die endständige Oberfläche des Verschlusskörpers mittels des Tubus an der Oberfläche des Verschlusskörpers außerhalb des zweiten Fluidkanals zumindest soweit bewirkt wird, dass eine Vergrößerung der Querschnittsfläche des zweiten Fluidkanals zumindest auf das 1,1, insbesondere 1,5, insbesondere 2, fache der Querschnittsfläche des zweiten Fluidkanals im undeformierten Zustand des Verschlusskörpers bewirkt wird. Dabei wird vorteilhafterweise der Verschlusskörper von seinem deformierten Zustand im Ruhezustand des Ventils direkt in einen dilatierten Zustand übergehen, ohne dabei in den undeformierten Zustand übergegangen zu sein.

Durch einen solchen geöffneten zweiten Fluidkanal im undeformierten Zustand des Verschlusskörpers werden das Verhalten und vor allem die Formstabilität des Verschlusskörpers besonders deutlich verbessert, insbesondere gegenüber Verschlusskörpern mit Schlitzanordnungen. Durch einen erfindungsgemäßen zweiten Fluidkanal wird unter der Einführung des Tubus durch die Ventilöffnung in den Öffnungskanal weniger mechanische Spannung auf dem Verschlusskörper, insbesondere auch bei der Dilatation des zweiten Fluidkanals, ausgeübt, wodurch, insbesondere bei mehrmaligem Öffnen und Schließen des Ventils bzw. Einführen und Entfernen des Tubus, das Verschlusskörpermaterial weniger stark beansprucht wird, deshalb weniger wahrscheinlich Rissbildungen des Verschlusskörpers, insbesondere an bzw. in der Nähe des zweiten Fluidkanals, auftreten und dadurch ein Benutzten des Ventils länger zuverlässig möglich ist. Zudem ist das Ventil im Ruhezustand derart durch den Verschlusskörper, welcher mit seinem zweiten Abschnitt im Öffnungskanal angeordnet ist, verschlossen, dass im Ruhezustand kein Fluid durch den zweiten Abschnitt austreten kann. Der Verschlusskörper des Ventils wird beim Einführen des Tubus durch die Ventilöffnung in den Öffnungskanal so deformiert, dass der zweite Fluidkanal über seine Erstreckung, also Öffnungsweite, im undeformierten Zustand des Verschlusskörpers hinaus dilatiert wird. Dabei kann der zweite Fluidkanal sehr einfach und ohne weitere Hilfsmittel dilatiert, also erweitert werden, so dass ein effektiver, insbesondere ein schnellerer und/oder größerer und/oder sicherer, Fluidaustausch zwischen Tubus und Ventil ermöglicht wird, insbesondere gegenüber Ventilen aus dem Stand der Technik. Dabei wird die Sicherheit dadurch gewährleistet, dass das Ventil beim Entfernen des Tubus aus dem Öffnungskanal, insbesondere sicher und/oder automatisch und/oder effektiv, wieder in den Ruhezustand des Ventils gebracht wird, und somit wieder verschlossen ist. Besonders da die Dilatation des Verschlusskörpers eine Vergrößerung der Querschnittsfläche des zweiten Fluidkanals auf zumindest das 1,1-fache, also eine Querschnittsfläche mit 110%, der Querschnittsfläche des zweiten Fluidkanals im undeformierten Zustand des Verschlusskörpers, bewirkt, ist ein derartiger Fluidaustausch zwischen Tubus und Ventil gewährleistet, dass eine zuverlässige und sichere Hinzugabe und/oder Abgabe des Fluids möglich, auch wiederholt möglich ist. Dies ist mit vorbekannten Verschlusskörpern, die einen geöffneten Fluidkanal im undeformierten Zustand des Verschlusskörpers, und insbesondere keinen Schlitz, aufweisen, nicht realisierbar, da nicht gewährleistet ist, dass das Ventil zuverlässig wiederholt schließt und ausreichend weit öffnet, keine bzw. kaum Materialbeschädigungen bei wiederholtem Einführen und Entfernen des Tubus und effektiver bzw. großer Fluidaustausch möglich ist.

Vorteilhafterweise ist das Ventil in erster Linie ein Objekt mit größerer Längserstreckung als Quererstreckung und mit Ventilöffnung an einem Ende der Längserstreckung und eine am anderen Ende der Längserstreckung angeordneten eine Fluidöffnung, die insbesondere dazu eingerichtet ist, eine Verbindung zum Fluidtransport mit einem an das Ventil angeschlossenen Anschlussstück oder Volumen herzustellen.

Vorteilhafterweise ist das Ventil so eingerichtet ist, dass ausgehend vom offenen Zustand beim Entfernen des Tubus aus dem Öffnungskanal, und insbesondere der Ventilöffnung, der zweite Abschnitt so in den Öffnungskanal zurückdrängt wird, dass der Ruhezustand hergestellt wird. Insbesondere wird dies allein durch den Verschlusskörper, insbesondere ein teilweises Entspannen des in Längsrichtung komprimierten Verschlusskörpers, bewirkt.

Vorteilhafterweise besteht der Verschlusskörper aus einem Polymer, insbesondere einem für medizinische Zwecke sterilem Polymer, insbesondere einem nicht vulkanisierten Polymer, insbesondere aus Silikon, zu mindestens 60%, insbesondere mindestens 80%, insbesondere aus Flüssig-Silikon (LSR).

Vorteilhafterweise wird und/oder ist der Verschlusskörper in einem einzigen Herstellungsprozess geformt, insbesondere aus den benötigten Rohmaterialien gegossen oder gespritzt, wobei insbesondere zumindest ein erster und zweiter Fluidkanal innerhalb des Verschlusskörpers, insbesondere mindestens der erster und der zweiter Fluidkanal, insbesondere zusammen über die gesamte Längserstreckung des Verschlusskörpers, während des Formens des Verschlusskörpers ausgebildet und/oder ausgespart wird. Ein solches Verfahren vereinfacht die Herstellung des Verschlusskörpers, wobei zugleich gewährleistet wird, dass sowohl der Verschlusskörper als auch die darin angeordneten Fluidkanäle bei allen so hergestellten Verschlusskörpern relativ gleiche vorteilhafte Merkmale und Dimensionen aufweisen.

Vorteilhafterweise ist der Ventilkörper in sich steif, insbesondere unelastisch, ausgebildet. Dabei weist ein solcher in sich steifer Ventilkörper insbesondere eine Steifigkeit des Materials auf, die durch ein Elastizitätsmodul gekennzeichnet ist, das einen Wert von mehr als 1 GPa aufweist und/oder größer ist als das des Verschlusskörpers.

Das Ventil ist vorteilhafterweise so eingerichtet, dass unter Einführung des Tubus durch die Ventilöffnung in den Öffnungskanal das Ventil aus seiner Ruhelage in den offenen Zustand gebracht wird. Vorteilhafterweise besitzt der Tubus dabei einen Außendurchmesser, der kleiner ist als der Durchmesser der Ventilöffnung und größer ist als 50% des Durchmessers der Ventilöffnung, wobei bevorzugt der Tubus einen Innendurchmesser des Tubus im Querschnitt des Tubus aufweist, der größer ist als 25% des Durchmessers der Ventilöffnung und kleiner ist als der Durchmesser der Ventilöffnung. Insbesondere ist der Tubus ein Luer Konus.

Vorteilhafterweise ist der Verschlusskörper und/oder der Ventilkörper so eingerichtet und/oder wird das Verschließen so durchgeführt, dass im Ruhezustand des Ventils der zweite Fluidkanal bis zu einem Druck, insbesondere bis zu einer Differenz des Innendrucks innerhalb des Verschlusskörpers aufgrund einer darin befindlichen Flüssigkeit zu einem Außendruck, von mehr als 0,1 MPa, insbesondere mehr als 0,2 MPa, geschlossen bleibt.

Vorteilhafterweise ist und/oder wird der erste Fluidkanal in einem Ruhezustand des Ventils, in dem das Ventil durch den Verschlusskörper verschlossen ist und/oder wird, mit der endständigen Fluidöffnung und/oder dem endständigen Fluidkanal des Ventils verbunden und/oder ist der zweite Fluidkanal gegenüber dem ersten Fluidkanal verjüngt, und/oder sind insbesondere zumindest eine Mehrzahl von Hohlraumabschnitten des Verschlusskörpers, insbesondere des ersten Fluidkanals, derart ausgebildet, dass zumindest ein sich verjüngender und/oder zumindest ein kleiner und zumindest ein größerer Hohlraumabschnitt, insbesondere ein kleinerer oder verjüngter Fluidkanal zwischen zwei vergrößerten Hohlraumabschnitten, insbesondere die Hohlraumabschnitte Sanduhr artig, ausgebildet sind. Durch eine solche geometrische Ausformung des Hohlraums des Verschlusskörpers, insbesondere des ersten Fluidkanals, wird bewirkt, dass nach der Deformation des Verschlusskörpers durch das Einführen des Tubus in das Ventil bei der Entfernung des Tubus aus dem Ventil ein Unterdruck entsteht, sodass kaum bzw. kein Rest eines Fluids an dem Tubus nach der Entfernung des Tubus aus dem Ventil verbleibt. Ebenso wird durch eine solche geometrische Ausformung des Verschlusskörpers, insbesondere des ersten Fluidkanals, insbesondere raschen Zugabe oder Abgabe, von Fluid in das/aus dem geöffnete(n) Ventil durch den Tubus die Innenfläche des Ventils, insbesondere die Innenseiten des Verschlusskörpers, effektiver gereinigt. Gemäß der Erfindung ist das Ventil so ausgebildet oder wird das Verfahren so durchgeführt, dass die Dilatation des zweiten Fluidkanals unmittelbar und allein durch die Krafteinwirkung des Tubus auf die endständige Oberfläche des Verschlusskörpers außerhalb des zweiten Fluidkanals zumindest soweit bewirkt wird, dass eine Vergrößerung der Querschnittsfläche des zweiten Fluidkanals zumindest auf das 1,1 fache der Querschnittsfläche des zweiten Fluidkanals im undeformierten Zustand des Verschlusskörpers bewirkt wird. Dabei ist unmittelbar und allein durch die Krafteinwirkung des Tubus insbesondere so zu verstehen, dass durch die Krafteinwirkung durch den Kontakt des Tubus mit der endständigen Oberfläche auf die endständige Oberfläche diese Dilatation bewirkt wird, ohne dass für die Dilatation des zweiten Fluidkanals, insbesondere abgesehen von einer Gegenkraft durch eine Lagerung und/oder Festlegung des Verschlusskörpers im Ventilkörper, eine weitere Kraft benötigt wird und ohne dass die Kraft über ein weiteres Element übertragen werden müsste.

Somit lässt sich der Aufbau des Ventils derart vereinfachen, dass keine weiteren Teile bzw. Gegenstände notwendig und/oder vorgesehen sind, um das Ventil wiederholt zu öffnen und zu schließen. Insbesondere wird dadurch der Verschlusskörper, abgesehen von einer Lagerung und/oder Festlegung im Ventilkörper nur an einer Fläche mit Gegenständen in Kontakt gebracht, die eine öffnende Kraft auf ihn auswirken, was eine Ermüdungserscheinung und somit einen Verschleiß des Verschlusskörpers bei wiederholtem Öffnen und Schließen des Ventils und wieder holten Einführen und Entfernen des Tubus in bzw. aus dem Ventil reduziert und die Robustheit des Ventils erhöht.

Vorteilhafterweise ist das Ventil dadurch gekennzeichnet und/oder wird der Abschnitt und/oder Verschlusskörper so verbracht und/oder angeordnet, dass der Verschlusskörper im Ruhezustand des Ventils nicht mehr als 5 mm, insbesondere weniger als 1 mm, insbesondere gar nicht, aus der Ventilöffnung und/oder aus dem Ventilkörper hervorsteht und/oder die endständige Oberfläche des Verschlusskörpers mit einem Winkel von weniger als 25°, insbesondere weniger als 5°, bevorzugt 0°, zu der Ebene, in der die Ventilöffnung im Ventilkörper liegt, ausgebildet ist. Auch durch eine solche Geometrie der endständigen Oberfläche und/oder Erstreckung des Verschlusskörpers ist im Ruhezustand des Ventils der zweite Fluidkanal hinreichend gut geschlossen und es bedarf keiner besonderen geometrischen Formen, insbesondere konkave oder konvexe, der endständigen Oberfläche des Verschlusskörpers oder einer längeren Erstreckung des Verschlusskörpers, so dass dieser wesentlich aus der Ventilöffnung hervorsteht. Dabei ist ein Hervorstehen des Verschlusskörpers aus der Ventilöffnung insbesondere derart zu verstehen, dass in einer Längserstreckung des Ventilkörpers und/oder des Ventils, der Ventilkörper keine Erstreckung in dessen Längserstreckung aufweist, die dazu führt, dass irgendein Abschnitt des Verschlusskörpers wesentlich, also mehr als 5 mm, insbesondere mehr als 1 mm, insbesondere gar nicht, außerhalb des Ventilkörpers bzw. des Ventils angeordnet ist. Ebenso wird durch eine derartige Geometrie und passgenaue Längserstreckung des Verschlusskörpers ein Reinigen, insbesondere ein Sterilisieren, der endständigen Oberfläche und des Ventils an sich erleichtert.

Vorteilhafterweise ist das Ventil dadurch gekennzeichnet oder wird das Schlie-ßen so durchgeführt, dass der zweite Abschnitt im Ruhezustand des Ventils eine Unter- oder Innenfläche insbesondere in das innere des Ventils oder des Ventilkörpers und/oder weg vom zweiten Fluidkanal gewandt und/oder der endständigen Oberfläche gegenüberliegend, ausbildet, an der der zweite Fluidkanal, insbesondere im inneren des Verschlusskörpers, mündet, die einen Winkel zu der Längserstreckung des Ventils und/oder Verschlusskörpers und/oder einer zur Ebene der Ventilöffnung und/oder endständigen Oberfläche senkrechten Ebene von mehr als 75° und weniger als 115°, insbesondere mehr als 85° und weniger als 95°, bevorzugt von 90°, aufweist und/oder parallel zur endständigen Oberfläche verläuft. Insbesondere grenzen an diese Unter- oder Innenfläche allseitig Wände, insbesondere des ersten Fluidkanals, in einem Winkel von 80 bis 100° an.

Eine solche, insbesondere relativ senkrecht zur Längserstreckung des Ventils und/oder Verschlusskörpers und/oder parallel zur Ebene der Ventilöffnung und/oder endständigen Oberfläche, angeordnete Unter- oder Innenfläche hat den Vorteil, dass der Verschlusskörper besonders einfach hergestellt werden kann, da keine besonderen Geometrien, wie zum Beispiel Zuspitzungen oder geringe Toleranzen bei deren Dimensionierung und Anordnung bei der Herstellung des Verschlusskörpers berücksichtigt werden müssen.

Vorteilhafterweise ist das Ventil so eingerichtet und/oder wird der Abschnitt und/oder Verschlusskörper so verbracht und/oder angeordnet und/oder das Verfahren so durchgeführt, dass im Ruhezustand des Ventils der zweite Abschnitt durch den Öffnungskanal so deformiert ist, dass der zweite Fluidkanal verschlossen ist, und/oder die Ventilöffnung und/oder der Öffnungskanal einen runden Innenquerschnitt aufweist und/oder die Ventilöffnung und/oder der Öffnungskanal in ihrem Querschnitt senkrecht zur Längserstreckung des Ventils und/oder Verschlusskörpers und/oder parallel zur Ebene der Ventilöffnung und/oder endständigen Oberfläche zumindest eine erste Erstreckung zwischen geometrischen Mittelpunkt des Querschnitts und einem Punkt auf dem Außenumfang des Querschnitts aufweist, die kleiner ist als zumindest eine zweite Erstreckung zwischen geometrischen Mittelpunkt des Querschnitts des zweiten Abschnitts senkrecht zur Längserstreckung des Ventils und/oder Verschlusskörpers und/oder parallel zur Ebene der Ventilöffnung und/oder endständigen Oberfläche und einem Punkt auf dem Außenumfang des Querschnitts des zweitens Abschnitts im undeformierten Zustand des Verschlusskörpers, wobei diese zumindest eine erste Erstreckung im Ruhezustand des Ventils mit der zumindest einen zweiten, jedoch komprimierten, Erstreckung zusammenfällt und/oder deckungsgleich ist Alternativ oder zusätzlich wird im Ruhezustand des Ventils vorzugsweise auf den zweiten Abschnitt aufgrund der Anordnung im Öffnungskanal und durch den Öffnungskanal eine radiale Kraft aufgebracht, die den zweiten Abschnitt komprimiert und/oder ist das Ventil ausgebildet, eine solche auszuüben. Insbesondere wird der zweite Abschnitt so deformiert und/oder komprimiert und/oder ist das Ventil entsprechend eingerichtet, dass allein dadurch der zweite Fluidkanal hinreichend verschlossen wird. Dabei sind keine weiteren Hilfsmittel, wie z. B. eine steifere Ummantelung um den Verschlusskörper herum, Zuspitzungen an der Unterseite des zweiten Abschnitts, konkave oder konvexe Geometrien der Ober- und/oder Unterseite, Verdickungen bzw. Flügel angebracht an dem Öffnungskanal, notwendig und insbesondere nicht vorhanden, um den zweiten Fluidkanal zu verschlissen, da dies durch den, insbesondere einen runden Innenquerschnitt aufweisenden, Öffnungskanal bewirkt wird.

Vorteilhafterweise ist das Ventil so ausgebildet und/oder wird der Abschnitt und/oder Verschlusskörper so verbracht und/oder angeordnet und/oder dilatiert und/oder das Verfahren so durchgeführt, dass in einem undeformierten Zustand des Verschlusskörpers der Außenumfang des zweiten Abschnitts in einem Querschnitt des zweiten Abschnitts so eingerichtet ist, dass die mindestens eine dritte Erstreckung zwischen dem geometrischen Schwerpunkt des Außenumfangs und einem ersten Punkt des Außenumfangs angeordnet ist, die kleiner ist als mindestens eine vierte Erstreckung zwischen dem geometrischen Schwerpunkt des Außenumfangs und einem zweiten Punkt des Außenumfangs, wobei insbesondere die erste Erstreckung und die zweite Erstreckung senkrecht zu einander stehen und/oder die vierte Erstreckung gleichzeitig die zweite Erstreckung darstellt und/oder insbesondere die Geometrie des Außenumfangs durch eine mindestens 2-zählige Drehachse durch den geometrischen Schwerpunkt des Außenumfangs, insbesondere in der Ebene des Außenumfangs und/oder senkrecht zur Ebene des Außenumfangs, verlaufend definiert wird, und insbesondere der Außenumfang eine Ellipse darstellt.

Vorteilhafterweise ist der erste Abschnitt des Verschlusskörpers im Querschnitt nicht rund, sondern insbesondere elliptisch oder oval ausgebildet und ist der Öffnungskanal im Querschnitt rund ausgebildet, wobei der Radius oder kleinste und/oder größte Radius des Öffnungskanals kleiner ist als der Radius des Umkreises eines Querschnitts, insbesondere aller Querschnitte, durch den ersten Abschnitt.

Erfindungsgemäß ist der zweite Abschnitt im Querschnitt so ausgebildet, dass seine Form zwei Hauptträgheitsachsen senkrecht zur Längserstreckung des Ventils und/oder Verschlusskörpers und/oder parallel zur Ebene der Ventilöffnung und/oder endständigen Oberfläche aufweist und entlang dieser Hauptträgheitsachsen unterschiedliche Erstreckungen aufweist.

Mit besonderem Vorteil ist der zweite Abschnitt so ausgebildet, dass seine Außenkontur im Querschnitt senkrecht zur Längserstreckung des Ventils und/oder Verschlusskörpers und/oder parallel zur Ebene der Ventilöffnung und/oder endständigen Oberfläche eine 2-zählige Drehachse durch den geometrischen Schwerpunkt und/oder genau zwei Spiegelebenen durch den geometrischen Schwerpunkt aufweist und er entlang dieser Spiegelebenen und/oder Achsen unterschiedliche Erstreckungen aufweist.

Durch eine solche Ausgestaltung, insbesondere nämlich im Grunde eine nicht runde bzw. kreisförmige Außengeometrie, eine des zweiten Abschnitts kann besonders einfach eine Deformation des zweiten Abschnitts erfolgen, wenn der Verschlusskörper im Ventil angeordnet ist, und der zweite Abschnitt im Öffnungskanal angeordnet ist, wobei dieser Öffnungskanal entsprechend einen runden Innenquerschnitt aufweist. Dadurch kann besonders vorteilhaft durch einen solch runden Öffnungskanal auf den zweiten Abschnitt eine den Verschlusskörper komprimierende bzw. deformierende Kraft wirken.

Erfindungsgemäß ist der zweite Fluidkanal so ausgebildet, dass seine Form zwei Hauptträgheitsachsen senkrecht zur Längserstreckung des Ventils und/oder Verschlusskörpers und/oder parallel zur Ebene der Ventilöffnung und/oder endständigen Oberfläche aufweist und entlang dieser Hauptträgheitsachsen unterschiedliche Erstreckungen aufweist, insbesondere um einen Faktor von mindestens drei, insbesondere mindestens vier und/oder maximal 5, unterschiedlich. Mit besonderem Vorteil ist der zweite Fluidkanal im Querschnitt nicht rund ausgebildet, insbesondere elliptisch oder oval.

Mit besonderem Vorteil ist der zweite Fluidkanal im Querschnitt so ausgebildet, dass seine Form im Querschnitt senkrecht zur des Ventils und/oder Verschlusskörpers und/oder parallel zur Ebene der Ventilöffnung und/oder endständigen Oberfläche eine 2-zählige Drehachse durch den geometrischen Schwerpunkt und/oder genau zwei Spiegelebenen durch den geometrischen Schwerpunkt aufweist und er entlang dieser Spiegelebenen und/oder Achsen unterschiedliche Erstreckungen aufweist, insbesondere um einen Faktor von mindestens drei, insbesondere mindestens vier und/oder maximal 5, unterschiedlich.

Mit besonderem Vorteil sind die Hauptträgheitsachsen und/oder Achsen und/oder Spiegelebenen der Außenkontur des zweiten Abschnitts senkrecht zueinander angeordnet und/oder sind die Hauptträgheitsachsen und/oder Achsen und/oder Spiegelebenen des zweiten Fluidkanal senkrecht zueinander angeordnet.

Erfindungsgemäß sind die Hauptträgheitsachsen des zweiten Abschnitts und die Hauptträgheitsachsen des zweiten Fluidkanal so angeordnet, dass die Hauptträgheitsachsen des zweiten Abschnitts zu den Hauptträgheitsachsen des zweiten Fluidkanals Winkel kleiner 20° einschließen. Mit besonderem Vorteil sind die Achsen und/oder Spiegelebenen des zweiten Abschnitts und die Hauptaschen, Achsen und/oder Spiegelebenen des zweiten Fluidkanal so angeordnet, dass sie deckungsgleich und/oder parallel sind und/oder die Hauptachsen, Achsen und/oder Spiegelebenen des zweiten Abschnitts zu den Hauptachsen, Achsen und/oder Spiegelebenen des zweiten Fluidkanals Winkel kleiner 20° einschließen, insbesondere in einer Projektion entlang der Längserstreckung des Ventils und/oder Verschlusskörpers und/oder senkrecht zur endständigen Oberfläche und/oder senkrecht zur Ebene der Ventilöffnung, wobei der Winkel insbesondere von jeder Richtung ausgehend zu der Richtungen mit den kleinsten Winkelabstand gemessen werden. Mit besonderem Vorteil ist die Hauptachse, Achse, Hauptträgheitsachse und/oder Spiegelebene des zweiten Fluidkanals, in deren Richtung der zweite Fluidkanal seine kleinere Erstreckung aufweist deckungsgleich, parallel oder mit einem Winkel kleiner 20° zu der Hauptachse, Achse, Hauptträgheitsachse und/oder Spiegelebene des zweiten Abschnitts, in deren Richtung der zweite Abschnitt seine größere Erstreckung aufweist. Mit besonderem Vorteil ist die Hauptasche, Achse, Hauptträgheitsachse und/oder Spiegelebene des zweiten Fluidkanals, in deren Richtung der zweite Fluidkanal seine größere Erstreckung aufweist deckungsgleich, parallel oder mit einem Winkel kleiner 20° zu der Hauptasche, Achse, Hauptträgheitsachse und/oder Spiegelebene des zweiten Abschnitts, in deren Richtung der zweite Abschnitt seine kleinere Erstreckung aufweist.

Insbesondere weist der erste Abschnitt eine Länge zwischen 2 und 4 mm auf. Insbesondere verjüngt sich der Verschlusskörper von der endständigen Oberfläche und/oder dem ersten Abschnitt zum zweiten Abschnitt hin, insbesondere nach einer anfänglichen Verbreiterung, um sich anschließend wieder zu verbreitern, um sich vorteilhafterweise erneut zu verjüngen und wieder zu verbreitern, insbesondere auf die Breite seines der endständigen Oberfläche und/oder dem ersten Abschnitt gegenüberliegenden Ende.

Mit besonderem Vorteil weist der zweite Fluidkanal eine kürze Erstreckung senkrecht zur endständigen Oberfläche und/oder senkrecht zur Ebene der Ventilöffnung und/oder in Richtung der Längserstreckung des Ventils und/oder Verschlusskörpers und/oder auf als der erste Fluidkanal.

Mit besonderem Vorteil wird das Verfahren so durchgeführt, dass und/oder ist der erste Fluidkanal im undeformierten Zustand des Verschlusskörpers im Querschnitt, insbesondere allen Querschnitten mindestens viermal so groß wie der zweite Fluidkanal im undeformierten Zustand. Insbesondere verjüngt sich der erste Fluidkanal im undeformierten Zustand von einem an den zweiten Fluidkanal angrenzenden Ende des ersten Fluidkanals ausgehend, insbesondere um sich im weiteren Verlauf zu einem vom zweiten Fluidkanal abgewandten Ende wieder zu verbreitern.

Insbesondere ist und/oder wird der Verschlusskörper, insbesondere nur sein zweiter Abschnitt und/oder abgesehen von seinem ersten Abschnitt, im Ruhezustand des Ventils in seiner Längserstreckung komprimiert angeordnet, insbesondere durch einem Boden des Ventilkörpers und einer Schulter, die insbesondere an das in das Innere des Ventilkörpers gerichtete Ende des Öffnungskanals anschließt.

Insbesondere ist und/oder wird nur der zweite Abschnitt des Verschlusskörpers im Ruhezustand des Ventils radial komprimiert und/oder ist der erste Abschnitt des Verschlusskörpers im Ruhezustand des Ventils nicht radial komprimiert.

Insbesondere besteht der Verschlusskörper ausschließlich aus dem ersten und dem zweiten Abschnitt und/oder erstreckt sich der erste Fluidkanal über die gesamte Längserstreckung des ersten Abschnitts, die insbesondere eine Länge zwischen 5 und 15 mm aufweist, und/oder erstreckt sich der zweite Fluidkanal über die gesamte Erstreckung des zweiten Abschnitts in Richtung der Längserstreckung des ersten Abschnitts, des Ventils und/oder Verschlusskörpers und/oder senkrecht zur Ebene der Ventilöffnung und/oder endständigen Oberfläche, die insbesondere eine Länge zwischen 0,3 und 3 mm, insbesondere zwischen 1 und 3 mm, aufweist.

Mit besonderem Vorteil weist der Verschlusskörper abgesehen von dem ersten und zweiten Fluidkanal keine Hohlräume auf. Mit besonderem Vorteil erstrecken sich der erste und/oder zweite Fluidkanal geradlinig, gegebenenfalls mit Verjüngungen und Erweiterungen, aber insbesondere ohne Kurve, insbesondere entlang einer gemeinsamen Geraden, die insbesondere die Achse der Längserstreckung des Verschlusskörpers ist. Insbesondere ist im geöffneten Zustand des Ventils zumindest ein gerader Fluidpassagepfad von der Ventilöffnung bis zur endständigen Fluidöffnung, insbesondere ein rundrohrförmiger gerader Fluidpassagepfad, insbesondere mit einem Radius von mindestens 0,9mm, insbesondere von mindestens 1 mm.

Mit besonderem Vorteil ist der Verschlusskörper einstückig, insbesondere in einem Guss und/oder in einer Spritzung und/oder aus nur einem Material gebildet. Insbesondere ist er ausschließlich durch Guss und/oder Spritzung hergestellt.

Insbesondere besteht das Ventil maximal oder nur aus dem Ventilkörper und dem Verschlusskörper und Klebstoff, insbesondere maximal aus dem Ventilkörper und dem Verschlusskörper, wobei der Ventilkörper insbesondere aus maximal vier, insbesondere maximal zwei Teilen gebildet, die miteinander verschweißt und/oder verklebt sind.

Mit Vorteil weist der Öffnungskanal in Richtung der Längserstreckung des Verschlusskörpers und/oder des Ventils und/oder endständigen Oberfläche und/oder senkrecht zur der Ebene, in der die Ventilöffnung liegt, eine Erstreckung von 1,5 bis 3,5 mm auf und/oder eine Quererstreckung und/oder einen Durchmesser zwischen 3,5mm und 5,5 mm, insbesondere zwischen 4 und 4,5mm, auf.

Mit besonderem Vorteil weist der breitere Kanal eine maximale Breite oder einen maximalen Durchmesser zwischen 5,8mm und 13 mm, insbesondere zwischen 7,5 und 11 mm, auf, insbesondere weist er einen Durchmesser zwischen 5,8mm und 13 mm, insbesondere zwischen 7,5 und 11 mm, über eine Länge zwischen 3 und 9 mm, insbesondere zwischen 3 und 7 mm, auf.

Alternativ jedoch nicht beansprucht ist es, wenn die Ventilöffnung und/oder der Öffnungskanal einen Innenumfang definiert, wobei in einer Aufsicht auf die die Ventilöffnung und/oder den Öffnungskanal, insbesondere entlang der Längserstreckung des Ventils und/oder Verschlusskörpers, dieser Innenumfang so eingerichtet ist, dass er mindestens eine Erstreckung zwischen geometrischen Schwerpunkt des Innenumfangs der Ventilöffnung und/oder des Öffnungskanals und einem ersten Punkt des Innenumfangs der Ventilöffnung und/oder des Öffnungskanals aufweist, die kleiner ist als mindestens eine größere Erstreckung zwischen dem geometrischen Schwerpunkt des Innenumfangs der Ventilöffnung und/oder des Öffnungskanals und einem zweiten Punkt des Innenumfangs der Ventilöffnung und/oder des Öffnungskanals. Dabei weist der zweite Abschnitt ein Außenumfang auf, der einem Kreis entspricht. Wobei der Radius des Kreises größer ist als die Erstreckung zwischen geometrischen Schwerpunkt des Innenumfangs der Ventilöffnung und/oder des Öffnungskanals und dem ersten Punkt.

Mit besonderem Vorteil weist das Ventil ein Luer-Lock-Konnektor, männlich oder weiblich, auf und/oder ist es mit einem solchen strömungstechnisch verbunden, insbesondere so, dass der erste Fluidkanal und/oder die Fluidöffnung so mit dem Luer-Lock-Konnektor strömungstechnisch verbunden ist, dass Flüssigkeit und/oder Gas vom Luer-Lock-Konnektor, insbesondere ungehindert, zum ersten Fluidkanal und/oder zur Fluidöffnung gelangen kann und/oder Flüssigkeit und/oder Gas vom ersten Fluidkanal und/oder zur Fluidöffnung, insbesondere ungehindert, zum Luer-Lock-Konnektor gelangen kann.

Vorteilhafterweise wird das Verfahren so durchgeführt und/oder ist der Verschlusskörper so ausgebildet, dass dieser einen Querschnitt des zweiten Fluidkanals in einem undeformierten Zustand des Verschlusskörpers aufweist, wobei eine kleinste Achse des Querschnitts eine Länge von mindestens 0,3 mm und/oder weniger als 1 mm, insbesondere zwischen 0,5 und 0,7 mm, aufweist, und insbesondere weist der Querschnitt des zweiten Fluidkanals in dem undeformierten Zustand eine Fläche von mehr als 0.2 mm² und/oder weniger als 3.5 mm², insbesondere von 1 bis 1,5 mm², auf. Vorteilhafterweise weist der zweite Fluidkanals im undeformierten Zustand des Verschlusskörpers einen Querschnitt auf, wobei eine kleinste Achse des Querschnitts eine Länge von mindestens 5% und/oder weniger als 25% der Querschnittserstreckung der Ventilöffnung und/oder weist die Querschnittsachsenlänge des zweiten Fluidkanals im geöffneten Zustand des Ventils eine Erstreckung von 10 bis 20%, insbesondere von 14% bis 15%, der Querschnittserstreckung der Ventilöffnung Insbesondere weist der Querschnitt des zweiten Fluidkanals in dem undeformierten Zustand des Verschlusskörpers eine Fläche von mehr als 5% und/oder weniger als 25% der Querschnittsfläche der Ventilöffnung und/oder der Querschnittsfläche des zweiten Fluidkanals im geöffneten Zustand des Ventils, insbesondere von 9% bis 14% der Querschnittsfläche der Ventilöffnung und/oder der Querschnittsfläche des zweiten Fluidkanals im geöffneten Zustand des Ventils, auf. Vorteilhafterweise wird das Verfahren so durchgeführt und/oder ist der Verschlusskörper so ausgebildet, dass dieser in einem undeformierten Zustand des Verschlusskörpers in einem Querschnitt einen solchen Außenumfang des zweiten Fluidkanals aufweist, dass mindestens eine Erstreckung zwischen dem geometrischen Schwerpunkt des Außenumfangs und einem ersten Punkt des Außenumfangs, die kleiner ist als mindestens eine Erstreckung zwischen dem geometrischen Schwerpunkt des Außenumfangs und einem zweiten Punkt des Außenumfangs, wobei insbesondere die Geometrie des Außenumfangs durch eine mindestens 2-zählige Drehachse durch den geometrischen Schwerpunkt des Außenumfangs, insbesondere in der Ebene des Außenumfangs und/oder senkrecht zur Ebene des Außenumfangs, verlaufend definiert wird, und insbesondere der Außenumfang eine Ellipse darstellt. Vorteilhafterweise ist in dem undeformierten Zustand des Verschlusskörpers die Richtung der mindestens einen Erstreckung zwischen dem geometrischen Schwerpunkt des Außenumfangs und einem ersten Punkt des Außenumfangs senkrecht zu der Richtung der größten Erstreckung des zweiten Fluidkanals im Querschnitt ist.

Vorteilhafterweise ist der Verschlusskörper so ausgebildet, dass der erste Abschnitt einen endständigen Teilabschnitt aufweist, wobei der erste Fluidkanal, und insbesondere nicht der zweite Fluidkanal, an diesem mündet, und/oder dieser gegenüberliegend zum zweiten Abschnitt angeordnet ist, und/oder durch ein Zwischenteilabschnitt des ersten Abschnitts vom zweiten Abschnitt getrennt ist, und insbesondere an der endständigen Fluidöffnung und/oder und oder dem endständigen Fluidkanal des Ventils zumindest teilweise anliegt. Mit Vorteil weist der endständige Teilabschnitt die größte Breite, die größte Querschnittsfläche und/oder den größten Umfang des Verschlusskörpers auf. Mit Vorteil weist der Teilabschnitt mindestens ein Rastmittel und/oder eine Nut auf. Mit Vorteil ist das das Ventil so ausgebildet ist, dass der Teilabschnitts im Ruhezustand, im geöffneten Zustand des Ventils und in allen Zuständen beim Öffnen des Ventils aus dem Ruhezustand heraus und in allen Zuständen beim Überführen vom geöffneten Zustand in den Ruhezustand der endständige Teilabschnitt auf einem Boden des Ventilgehäuses aufliegt, wobei sich insbesondere vom Boden aus der endständige Fluidkanal zur endständigen Fluidöffnung erstreckt oder die endständigen Fluidöffnung im Boden angeordnet ist und/oder der Boden auch eine Begrenzung des breiten Kanals des Ventilkörpers darstellt und/oder wobei
der endständige Teilabschnitt eine Bodenfläche des Verschlusskörpers definiert, die im Ruhezustand des Ventils in einer ersten Lage oder Ebene angeordnet ist und im geöffneten Zustand des Ventils in einer zweiten Ebene oder Lage angeordnet ist, wobei die erste Lage oder Ebene und die zweite Lage oder Ebene um weniger als 5% der Längserstreckung des Verschlusskörpers in Längserstreckung des Verschlusskörpers verschoben sind, wobei die erste und die zweite Ebene insbesondere parallel zueinander und/oder senkrecht zur Längserstreckung des Ventils und/oder des Verschlusskörpers und/oder parallel zu der Ebene, in der die Ventilöffnung liegt, liegen.

Vorteilhafterweise wird das Verfahren so durchgeführt und/oder ist der Verschlusskörper so ausgebildet, dass im geöffneten Zustand des Ventils durch Einführung des Tubus durch die Ventilöffnung, insbesondere nur bis maximal zum Ende des Öffnungskanals und/oder bis zum Beginn des breiteren Kanals, eine Strecke auf dem zweiten Abschnitt, insbesondere der endständigen Oberfläche, die sich zwischen einem Punkt auf dem Außenumfang des zweiten Fluidkanals und einem Punkt auf dem Außenumfang des zweiten Abschnitts erstreckt, die einen Schnittwinkel (γ) zu einer Ebene, die parallel zur Ebene der Ventilöffnung im Ventilkörper und/oder senkrecht zur Längserstreckung des zweiten Fluidkanals und/oder des Ventils, aufweist, der größer als 5° und/oder kleiner als 355° ist.

Vorteilhafterweise wird das Verfahren so durchgeführt und/oder ist der Verschlusskörper so ausgebildet, dass im geöffneten Zustand des Ventils durch Einführung des Tubus durch die Ventilöffnung, insbesondere nur bis maximal zum Ende des Öffnungskanals und/oder bis zum Beginn des breiteren Kanals, eine Strecke auf dem zweiten Abschnitt, insbesondere auf der der endständigen Oberfläche abgewandten Seite des zweiten Abschnitts, insbesondere auf einer zur endständigen Oberfläche parallelen Ebene, an der der zweite Fluidkanal, insbesondere zum ersten Fluidkanal hin, mündet, die sich zwischen einem Punkt auf dem Außenumfang des zweiten Fluidkanals und einem Punkt auf dem Außenumfang des zweiten Abschnitts erstreckt, die einen Schnittwinkel (γ) zu einer Ebene, die parallel zur Ebene der Ventilöffnung im Ventilkörper und/oder senkrecht zur Längserstreckung des ersten Fluidkanals und/oder des Verschlusskörpers und/oder Ventils, aufweist, der größer als 5° und/oder kleiner als 355° ist.

Vorteilhafterweise wird das Verfahren so durchgeführt und/oder ist das Ventil so eingerichtet, dass im offenen Zustand des Ventils unter Einführung des Tubus durch die Ventilöffnung der zweite Abschnitt mindestens eine Strecke, die sich zwischen einem, auf der endständigen Oberfläche befindlichen, Punkt auf dem Außenumfang des zweiten Fluidkanals und einem Punkt auf dem Außenumfang der endständigen Oberfläche erstreckt, aufweist, die einen Schnittwinkel (γ) zu einer Ebene, die parallel zur Ventilöffnungsebene ist, aufweist, der größer als 5° und/oder kleiner als 355° ist.

Vorteilhafterweise wird das Verfahren so durchgeführt und/oder ist der Verschlusskörper so eingerichtet, dass im offenen Zustand des Ventils ein erster Schnittwinkel (α) zwischen einer ersten Geradenprojektion mindestens einer ersten Strecke, die sich zwischen einem, sich insbesondere auf der endständigen Oberfläche oder einer dieser gegenüberliegenden Oberfläche befindlichen, ersten Punkt auf dem Außenumfang des zweiten Fluidkanals und einem, sich insbesondere auf einer der Ventilöffnung zugewandten und/oder abgewandten Seite befindlichen, ersten Punkt auf dem Außenumfang des zweiten Abschnitts erstreckt, und einer zweiten Geradeprojektion mindestens einer, sich insbesondere auf der endständigen Oberfläche oder einer dieser gegenüberliegenden Oberfläche befindlichen, zweiten Strecke, die der ersten Strecke durch den Schwerpunkt des Außenumfangs des zweiten Fluidkanals getrennt und/oder, insbesondere diametral, gegenüberliegt, definiert ist und in dem Ruhezustand des Ventils ein zweiter Schnittwinkel (β) zwischen einer dritten Geradeprojektion mindestens einer dritten Strecke, die sich zwischen dem ersten Punkt auf dem Außenumfang des zweiten Fluidkanals und dem ersten Punkt auf dem Außenumfang des zweiten Abschnitts erstreckt, und einer vierten Geradenprojektion mindestens einer vierten Strecke, die der dritten Strecke durch den Schwerpunkt des Außenumfangs des zweiten Fluidkanals getrennt gegenüberliegt, definiert ist, wobei das Ventil so eingerichtet ist, dass der Unterschied zwischen dem ersten Schnittwinkel (α) und dem zweiten Schnittwinkel (β) mindestens 5°, insbesondere mindesten 15°, und/oder maximal 110°, insbesondere maximal 90°, beträgt.

Durch eine solche Deformation und dadurch bewirkte Änderung der Lage der Strecken bzw. der endständigen Oberfläche wird besonders vorteilhaft bewirkt, dass der Tubus nicht nur durch die Ventilöffnung eingeführt wird und den zweiten Abschnitt in einen breiteren Bereich verschiebt, sondern darüber hinaus, der zweite Fluidkanal derart dilatiert wird, dass der Fluidkanal eine breitere Öffnungsweite aufweist als in dem undeformierten Zustand des Verschlusskörpers, und insbesondere der Tubus dadurch weiter in das Ventil eingeführt werden kann und dabei besonders in den Fluidkanal ohne Ausüben von radial weitenden Kräften im Fluidkanal eingeführt werden kann und/oder wird. Dadurch wird ein besonders effizienterer Fluidaustausch ermöglicht.

Eine Gerade, die parallel zu oder in einer Ebene liegt, wird hier insbesondere als eine Gerade mit einem Winkel zu dieser Ebene von 0° bzw. 360° verstanden und nicht als solche mit einem Winkel von 180°.

Unter einen Geradenprojektion, auch Projektionsgerade wird insbesondere verstanden von einer Strecke eine Projektion des der Strecke innewohnenden Vektors, wodurch aus der Strecke eine Gerade im mathematischen Sinne wird, und somit sich die Geradenprojektionen von aufeinanderzulaufende Strecken, die aber räumlich durch durch einen Raum, wie z. B. eine Öffnung, getrennt sind, schneiden und somit einen Schnittwinkel bilden. Mathematisch wird demnach aus einer Strecke [AB] ein Vektor A̅B̅.

Vorteilhafterweise wird das Verfahren so durchgeführt und/oder ist der der Verschlusskörper mit solchen Verzerrungstensoren, die der Verschlusskörper in seinen Abschnitten aufweist, eingerichtet, dass unter Einführung des Tubus durch die Ventilöffnung in den Öffnungskanal und/oder den größeren Kanal des Ventilkörpers der zweite Abschnitt unter der Stauchung des ersten Abschnitts in den größeren Kanal des Ventilkörpers verschiebbar ist und/oder die endständige Oberfläche eine Änderung der Orientierung dieser Oberfläche zu einer senkrecht zur Längserstreckung des Ventils und/oder Verschlusskörpers und/oder parallel zur Ebene der Ventilöffnung verlaufenden Ebene um mehr als 5° mittels einer Scherung eines Abschnitts des Verschlusskörpers, insbesondere des ersten und/oder zweiten Abschnitts, erfährt und/oder die endständige Oberfläche eine Änderung der Orientierung dieser Oberfläche zu einer senkrecht zur Längserstreckung des Ventils und/oder Verschlusskörpers und/oder parallel zur Ebene der Ventilöffnung verlaufenden Ebene um mehr als 5° und eine Scherung eines Abschnitts des Verschlusskörpers durch eine relativ zu den anderen Abschnitten des Verschlusskörpers geringere Stauchung eines Zwischenteilabschnitts des ersten Abschnitts erfährt und/oder ein Anfangsteilabschnitt des ersten Abschnitts, der insbesondere an die endständige Oberfläche angrenzt oder diese ausbildet, durch einen Verzerrungstensor gekennzeichnet ist, der relativ zu seinem deviatorischen Anteil einen großen isotropen Anteil aufweist und, insbesondere einen relativ zu seinem deviatorischen Anteil größeren isotropen Anteil aufweist als der zweite Abschnitt und/oder der Rest des ersten Abschnitts.

Ein isotroper Anteil des Verzerrungstensors beschreibt insbesondere die Dehnung eines Teils und ein deviatorische Anteil des Verzerrungstensors insbesondere die Scherung eines Teils.

Vorteilhafterweise ist das Ventil und/oder der Verschlusskörper so ausgebildet oder wird das Verfahren so geführt, dass die endständige Oberfläche des Verschlusskörpers durch die Deformation des Verschlusskörpers und die Dilatation des zweiten Fluidkanals unter Einführung des Tubus durch die Ventilöffnung und den Öffnungskanal bis in den größeren Kanal des Ventilkörpers an der äußeren Mantelfläche des Tubus zumindest teilweise anliegt, insbesondere zu mindestens 50% der endständigen Oberfläche und/oder dass ein Punkt der endständigen Oberfläche, der an den zweiten Fluidkanal angrenzt, durch die Deformation des Verschlusskörpers und die Dilatation des zweiten Fluidkanals unter Einführung des Tubus durch die Ventilöffnung und den Öffnungskanal in den größeren Kanal des Ventilkörpers an der äußeren Mantelfläche des Tubus anliegt und/oder ein Zwischenteilabschnitt des ersten Abschnitts, wobei der Zwischenteilabschnitt zwischen einem endständigen Teilabschnitt des ersten Abschnitts und dem zweiten Abschnitt angeordnet ist, ein Einführen des Tubus durch die Ventilöffnung und den Öffnungskanal in den größeren Kanal des Ventilkörpers begrenzt und/oder
unter Einführung des Tubus durch die Ventilöffnung und den Öffnungskanal in den größeren Kanal des Ventilkörpers der erste Fluidkanal eine Verbindung zwischen einer endständigen Fluidöffnung des Ventils und dem Tubus herstellt. Durch diese Deformation des Verschlusskörpers und Dilatation des zweiten Fluidkanals, sodass mindestens 50% der endständigen Oberfläche und/oder ein Punkt der endständigen Oberfläche an der äußeren Mantelfläche des Tubus anliegt, wird die Öffnung des Tubus besonders wenig durch den Verschlusskörper verdeckt und somit ist ein besonders effizienter Fluidaustausch zwischen Tubus und Ventil möglich. Durch die vorteilhafte Weiterbildung des Zwischenteilabschnitts wird bewirkt, dass der Tubus nur so weit eingeführt wird, wie vorgesehen und daher durch das Einführen durch den Tubus keine Beschädigung des Ventils erfolgt, wodurch das Ventil eine längere Lebensdauer und häufigere Wiederbenutzung aufweist. Insbesondere wird durch ein solches Verschlussstück eine derartige Dilatation des zweiten Fluidkanals ermöglicht, dass der erste Fluidkanal eine Verbindung zwischen der Fluidöffnung und dem Tubus herstellt und somit der Tubus insbesondere in unmittelbarer Verbindung mit der Fluidöffnung des Ventils steht.

Vorteilhafterweise ist der Verschlusskörper dadurch gekennzeichnet, dass auf und/oder an dem Verschlusskörper eine Mehrzahl an Erhebungen, insbesondere Stege, angeordnet ist, die so eingerichtet sind, dass sie im Ruhezustand des Ventils am Ventilkörper anliegen und in den Bereich zwischen ihnen als Abstandshalter zwischen Ventilkörper und Verschlusskörper dienen.. Mit diesen Erhebungen kann verhindert werden, dass beim Einführen des Tubus ein zumindest ein Großteil des Verschlusskörpers an dem Ventilkörper anhaftet, somit sich der Verschlusskörper besser deformieren und/oder verschieben lässt und insbesondere bei der Einführung des Tubus nicht beschädigt wird und ein leichteres Einführen des Tubus und somit eine Bedienung mit geringerem Kraftaufwand ermöglicht wird.

Vorteilhafterweise wird das Verfahren so durchgeführt und/oder ist der Verschlusskörper und/oder der Ventilkörper so ausgebildet ist, dass im Ruhezustand des Ventils nicht der gesamte, Außenumfang des zweiten Abschnitts an der Innenseite des Ventilkörpers, , anliegt und/oder mindestens ein umlaufender Hohlraum zwischen Ventilkörper und Verschlusskörper und/oder zweitem Abschnitt vorliegt, insbesondere mit einer maximalen radialen Breite zwischen 0,6 und 1 mm und/oder einer maximalen Höhe zwischen 2 und 3 mm aufweist.

Vorteilhafterweise wird das Verfahren so durchgeführt und/oder ist der Verschlusskörper und/oder der Ventilkörper so ausgebildet ist, dass der Verschlusskörper, insbesondere der zweite Abschnitt und/oder der erste Abschnitt, im Ruhezustand des Ventils teilweise an der Innenfläche des Ventilkörpers, insbesondere der erste Abschnitt mit seiner Umfangsfläche vollständig an der Innenfläche des Öffnungskanals anliegt und der zweite Abschnitt teilweise an der Innenfläche des Ventilkörpers, insbesondere des Öffnungskanals und/oder des breiteren Kanals anliegt. Dabei ist der Vorteil, dass trotz des geringeren Materialeinsatzes bei dem Verschlusskörper, und somit kostengünstigerer Produktion, der Öffnungs- und Schließungsmechanismus des Ventils beim Einführen und Entfernen des Tubus aus bzw. in das Ventil optimal gewährleistet ist.

Vorteilhafterweise weist der Ventilkörper ein Gewinde auf, welches insbesondere ein sicheres Verbinden des Ventils mit einem Gegenstück zum Fluidaustausch zwischen Ventil und Gegenstück mittels einer endständigen Fluidöffnung des Ventils unterstützt. Insbesondere ist das Gewinde um den endständigen Fluidkanal und/oder die endständige Fluidöffnung angeordnet.

Vorteilhafterweise weist der Ventilkörper ein Gewinde auf, welches insbesondere ein sicheres Verbinden des Ventils mit einem Luer-Lock-Konus zum Fluidaustausch zwischen Ventil und Tubus unterstützt. Insbesondere ist das Gewinde um den Öffnungskanal und/oder die Ventilöffnung angeordnet.

Vorteilhafterweise weist der Ventilkörper auf Höhe des Öffnungskanals und/oder der Ventilöffnung auf einer Außenseite des Ventils Sicherungsmittel auf, die ein sicheres Einführen des Tubus unterstützen.

Bei Winkelangaben, wenn nicht weiter definiert, sind insbesondere Winkel zwischen Strecken und/oder Geraden und/oder Ebenen zu verstehen, die den spitzen Winkel darstellen und nicht den dazu komplementären stumpfen Winkel.

Vorteilhafterweise wird ein maximaler deformierter Zustand des Verschlusskörpers dadurch erreicht und/oder begrenzt und/oder definiert, dass der erste Abschnitt in den Zwischenabschnitt und/oder im Zwischenabschnitt verschoben und dort deformiert ist, und zumindest ein Abschnitt des Verschlusskörpers, insbesondere ein Abschnitt zwischen einem Endabschnitt, welche dem ersten endständigen Abschnitt endständig gegenüber liegt, und Zwischenabschnitt oder ein Abschnitt des Endabschnitt, ein weiteres Hineingeben des Tubus, insbesondere des Fluidkanals des Tubus, in das Ventil behindert.

Weiterhin offenbart ist auch ein Verfahren zum Offnen und Schließen eines Ventils mit einem, insbesondere nadellosen, Tubus, insbesondere Luer-Konus, wobei zum Schließen des Ventils ein Verschlusskörper des Ventils, der einen in einem Ruhezustand des Verschlusskörpers geöffneten Fluidkanal, insbesondere zweiten Fluidkanal, aufweist, oder Abschnitt, insbesondere zweiter Abschnitt, des Verschlusskörpers, in dem sich ein in einem Ruhezustand des Verschlusskörpers geöffneter Fluidkanal befindet, der an einer Verschlusskörperoberfläche mündet, in einen ersten Kanal, insbesondere Öffnungskanal, des Ventils, insbesondere eines Ventilkörpers des Ventils, verbracht wird, sodass der Verschlusskörper eine Öffnung des Ventilkörpers und/oder Ventils, insbesondere Ventilöffnung, verschließt, wobei der erste Kanal den Verschlusskörper oder den Abschnitt, insbesondere radial, komprimiert, sodass der Fluidkanal geschlossen wird und
wobei zum Öffnen der Verschlusskörper oder der Abschnitt durch Drücken mit dem Tubus auf den Verschlusskörper oder den Abschnitt, jedoch außerhalb des Fluidkanals, ohne Ausüben von radial weitenden Kräften im Fluidkanal in einen weiteren und/oder breiteren Kanal des Ventils, insbesondere Ventilkörpers, verbracht wird, der den Verschlusskörper oder den Abschnitt zumindest um 10% weniger, insbesondere nicht, komprimiert. Dabei werden anschließend durch weiteres Einführen des Tubus in das Ventil unter Drücken mit dem Tubus auf die Verschlusskörperoberfläche den Fluidkanal weitende, insbesondere radial weitende, Kräfte eingebracht und der Tubus in den Fluidkanal eingeführt.

Dabei weist der Verschlusskörper insbesondere auch einen ersten Abschnitt mit einem ersten Fluidkanal auf. Insbesondere ist der Verschlusskörper, der Ventilkörper, der Öffnungskanal, der erste Fluidkanal, der zweite Fluidkanal, der erste Abschnitt, der zweite Abschnitt, der weitere und/oder breitere Kanal ausgebildet wie oben ausgeführt und/oder wird das Verfahren durchgeführt wie oben als vorteilhaft ausgeführt.

Radial ist insbesondere als im Querschnitt auf ein Zentrum, insbesondere einen Punkt mit höchster Symmetrie und/oder auf der Längsachse, oder vom Zentrum weg zu verstehen. Eine radial komprimierende Kraft ist insbesondere derart zu verstehen, dass eine externe von außen wirkende radiale und komprimierende Kraft einwirkt, wobei die Kraft von außerhalb nicht zwingend, aber vorteilhafterweise von allen Richtungen innerhalb einer Querschnittsebene, also senkrecht zur Längserstreckung des Ventils und/oder Verschlusskörpers und/oder parallel zur Ebene der Ventilöffnung einwirkt. Eine radial dilatierende Kraft ist insbesondere derart zu verstehen, dass eine externe von innen wirkende radiale und dilatierende Kraft einwirkt, wobei die Kraft von innerhalb nicht zwingend, aber vorteilhafterweise von allen Richtungen innerhalb einer Querschnittsebene, also senkrecht zur Längserstreckung des Ventils und/oder Verschlusskörpers und/oder parallel zur Ebene der Ventilöffnung einwirkt. Bei der Verbringung des Verschlusskörpers oder des Abschnitts in den breiteren Kanal des Ventilkörpers übt dieser breitere Kanal eine geringere komprimierende Kraft auf den Verschlusskörper oder den Abschnitt aus. Dabei wirkt diese Kraft insbesondere ebenso radial auf den Verschlusskörper oder den Abschnitt. Die Kraft ist dabei um mindestens 10% geringer, insbesondere ist keine komprimierende Kraft vorhanden, als die Kraft die den Verschlusskörper oder den Abschnitt radial komprimiert, wenn der Verschlusskörper oder der Abschnitt in einem ersten Bereich des Ventils verbracht ist.

Vorteilhafterweise weist der geöffnete Fluidkanal in dem undeformierten Zustand des Verschlusskörpers eine im Querschnitt durch den Verschlusskörper in einer ersten Richtung eine Erstreckung von mindestens 0,3 mm und/oder maximal 0,9 mm, insbesondere zwischen 0,5 und 0,7 mm auf, und in einer zweiten zur ersten senkrechten Richtung eine Erstreckung von weniger als 4mm und/oder mehr als 1,8 mm, insbesondere von weniger als 3,5 und/oder mehr als 2,1mm,
der geöffnete Fluidkanal in dem undeformierten Zustand des Verschlusskörpers eine Querschnittsfläche von mehr als 0.2 mm² und/oder weniger als 3.5 mm², insbesondere zwischen 1 und 1,5 mm².

Vorteilhafterweise weist der geöffnete Fluidkanal in dem undeformierten Zustand des Verschlusskörpers eine Querschnittfläche aufweist, die in eine erste Richtung eine Länge von mindestens 5% und/oder weniger als 25%, insbesondere zwischen 10 und 20%, insbesondere zwischen 14% und 15%, der Querschnittserstreckung der Ventilöffnung in der ersten Richtung und/oder der Erstreckung des geöffneten Fluidkanals in die erste Richtung des Fluidkanals im geöffneten Zustand des Ventils, insbesondere von 10 bis 20%, insbesondere von 14% bis 15%, der Querschnittserstreckung der Öffnung des Ventils und/oder der Querschnittsachsenlänge des Fluidkanals im geöffneten Zustand des Ventils, auf, und ist insbesondere die erste Richtung diejenige, in der der Fluidkanal im undeformierten Ruhezustand des Verschlusskörpers im Querschnitt die kleinste Erstreckung aufweist und/oder insbesondere der Querschnitt des Fluidkanals in dem undeformierten Ruhezustand des Verschlusskörpers eine Querschnittsfläche von mehr als 5% und/oder weniger als 25%, insbesondere zwischen 9% und 14%, der Querschnittsfläche der Öffnung des Ventilkörpers und/oder der Querschnittsfläche des Fluidkanals im geöffneten Zustand des Ventils und/oder der Querschnittsfläche des Fluidkanals im geöffneten Zustand des Ventils, aufweist und/oder wird bei Einführen des Tubus der Fluidkanal derart radial geweitet, dass der Tubus so in das Ventil, insbesondere den Fluidkanal, eingeführt wird, dass mindestens 50% des Querschnitts des Innenkanals des Tubus innerhalb des Ventils, insbesondere des Fluidkanals als durch das Ventil, insbesondere gradlinig, durchgehender Kanal, freigegeben wird.

Vorteilhafterweise wird bei einem Verfahren zum Öffnen und Schließen eines Fluidkanals eines Ventils durch das Einführen des Tubus der geöffnete Fluidkanal so dilatiert, dass die kleinste Erstreckung des Querschnitts des Fluidkanals in dem Ruhezustand des Verschlusskörpers mindestens 5% und/oder weniger als 25%, insbesondere zwischen 10 und 20%, insbesondere zwischen 14% und 15% der Erstreckung der Öffnung des Ventils in einem parallelen Querschnitt in die selbe Richtung wie die kleinste Erstreckung und/oder der Erstreckung des Fluidkanals in einem parallelen Querschnitt in die selbe Richtung wie die kleinste Erstreckung im geöffneten Zustand des Ventils.

Vorteilhafterweise wird durch das Einführen des Tubus der geöffnete Fluidkanal so dilatiert, dass der Querschnitt des zweiten Fluidkanals in dem undeformierten Zustand eine Fläche von mehr als 5% und/oder weniger als 25%, insbesondere zwischen 9% und 14%, der Querschnittsfläche der Öffnung des Ventilkörpers und/oder der Querschnittsfläche des zweiten Fluidkanals im geöffneten Zustand des Ventils aufweist.

Vorteilhafterweise wird bei Einführen des Tubus der Fluidkanal derart radial geweitet wird, dass der Tubus so in das Ventil, insbesondere den Fluidkanal, eingeführt wird, dass mindestens 50% des Querschnitts des Innenkanals des Tubus innerhalb des Ventils, insbesondere des Fluidkanals, freigegeben wird.

Vorteilhafterweise wird beim Entfernen des Tubus durch teilweises Entspannen des Verschlusskörpers ein Unterdruck im Fluidkanal und/oder breiteren Kanal erzeugt.. Dies kann beispielsweise dadurch erreicht werden, dass der Fluidkanal mindesten einen ersten und zweiten Fluidkanal aufweist, wobei der erste Fluidkanal derart ausgebildet ist, dass zumindest ein verjüngter und zumindest ein größerer Hohlraumabschnitt, insbesondere ein verjüngter Fluidkanal zwischen zwei vergrößerten Hohlraumabschnitten, insbesondere die Hohlraumabschnitte Sanduhr artig, ausgebildet sind.

Mit besonderem Vorteil wird der Abschnitt und/oder Verschlusskörper durch ein teilweises Entspannen des in Längsrichtung komprimierten Verschlusskörpers in den ersten Kanal eines Ventilkörpers verbracht und/oder in dem ersten Kanal des Ventilkörpers angeordnet, insbesondere beim Entfernen eines eingeführten Tubus.

Weitere Vorteile und Ausgestaltungen sollen rein exemplarisch und nicht beschränkend an Hand der rein schematischen Figuren erläutert werden. Die Figuren zeigen:
- Figur 1:: eine Aufsicht auf einen Verschlusskörper im undeformierten Zustand des Verschlusskörpers;
- Figur 2:: eine Längsschnittansicht eines Ventils im Ruhezustand des Ventils, in dem das Ventil geschlossen ist;
- Figur 3:: eine Längsschnittansicht eines Ventils im offenen Zustand des Ventils und
- Figur 4:: eine Längsschnittansicht eines Ventils im offenen Zustand des Ventils, wobei der Tubus bis in den größeren Kanal des Ventilkörpers eingebracht wurde.

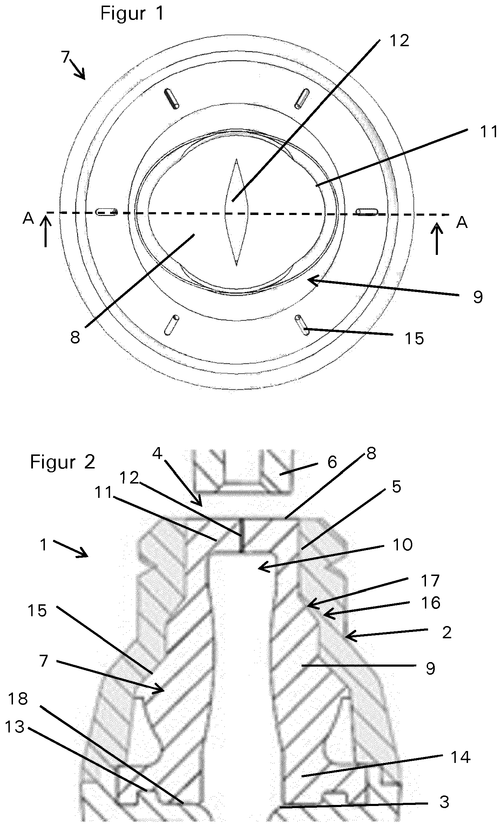

In Figur 1 ist der Verschlusskörper 7 in einer Aufsicht in seinem undeformierten Zustand dargestellt. Man blickt auf die endständige Oberfläche 8 des Verschlusskörpers 7, auf die erfindungsgemäß mit dem Tubus 6 bzw. der Unterseite des Tubus 6 ein Druck zum Öffnen des Ventils ausgeübt werden kann. In dem gezeigten undeformierten Zustand ist zu erkennen, dass der zweite Fluidkanal 12, der in einem zweiten und endständigen Abschnitt 11 des Verschlusskörpers 7 angeordnet ist und an der endständige Oberfläche 8 mündet, geöffnet ist. Zu erkennen ist, dass der zweite Fluidkanal 12 dabei keine runde Öffnung bildet, sondern annähernd eine Ellipse. Eine solche nicht runde Form hat sich als besonders günstig herausgestellt, um das sichere und wiederholbare Öffnen und Schließen des Ventils 1 zu ermöglichen. Zudem ist ersichtlich, dass der zweite Abschnitt 11 ebenso eine nicht runde Geometrie in seinem Querschnitt aufweist und sich an den ersten Abschnitt 9 anschließt. Der zweite Abschnitt 11 bildet dabei in seinem Querschnitt mit seiner Außenkontur eine elliptische Geometrie aus, wobei dessen Hauptachse senkrecht zur Hauptachse der elliptischen Geometrie des zweiten Fluidkanals 12 angeordnet ist. Eine solche Anordnung hat sich als besonders vorteilhaft erwiesen um ein sicheres Schließen des zweiten Fluidkanals 12 zu ermöglichen, wenn der Verschlusskörper in das Ventil 1 eingebaut ist.

Im ersten Abschnitt 9 ist dabei ein erster Fluidkanal 10 angeordnet, wobei der zweite Fluidkanal 12 verjüngt zu dem ersten Fluidkanal 10 ausgebildet und diese miteinander verbunden sind, weshalb in dieser Darstellung der Figur 1 der erste Fluidkanal 10 nicht gezeigt ist, da dieser unterhalb des zweiten Fluidkanals 12 liegt und er durch den zweiten Abschnitt 11 verdeckt wird.

Der Verschlusskörper 7 ist elastisch deformierbar ausgebildet, hier aus Silikon. Dies bedeutet, dass nach einer Deformation der Verschlusskörper 7 seine undeformierte Ursprungsform wieder einnimmt, wenn er keinen deformierenden Kräften mehr ausgesetzt ist.

Zu erkennen sind auch Antiklebestege 15, die ein Festkleben des Verschlusskörpers 7 an einem Ventilkörper, in den der Verschlusskörper 7 eingesetzt wird, verhindern.

In Figur 2 ist ein Längsschnitt durch ein erfindungsgemäßes Ventil 1 im Ruhezustand des Ventils 1 und ein Tubus 6 gezeigt. Dabei befindet sich der Tubus 6 nicht im Ventil 1, sondern außerhalb und ist nicht in Verbindung mit dem Ventil 1. Das Ventil weist einen Ventilkörper 2 auf, der um die Längsachse rotationssymmetrisch ausgebildet ist. Ein solcher Ventilkörper 2 stellt dabei ein wesentliches Gerüst des Ventils dar, welches in diesem Beispiel eines Ventils steif ausgebildet ist, und aus Hartplastik hergestellt wurde. Dabei kann der Ventilkörper die Außenseite des Ventils darstellen. Wie in der Figur 2 zu erkennen, ist der Ventilkörper 2 längs gestreckt, besitzt also eine größere Erstreckung in einer Länge als in seiner Breite. An einem Ende des Ventilkörpers befindet sich dabei eine Ventilöffnung 4. Diese Ventilöffnung 4 bildet somit eine Öffnung des Ventilkörpers und somit des Ventils. Durch diese Ventilöffnung 4 kann dabei ein Tubus 6 in das Ventil eingeführt werden. Dabei ist der Durchmesser der Ventilöffnung 4 so gewählt, dass er nur unwesentlich größer ist als der Außendurchmesser des Endes des Tubus 6. Durch solche Dimensionen wird gewährleistet, dass zum einen der Tubus 6 sicher in das Ventil 1 durch die Ventilöffnung 4 eingeführt werden kann, dabei aber geführt wird und seine Eindringtiefe begrenzt ist, der Tubus sich konisch weitend ausgeführt ist.. Die Ventilöffnung 4 grenzt dabei an einen endständigen Öffnungskanal 5 des Ventilkörpers 2 an. Um das Einführen des Tubus besonders zu begrenzen, ist der Öffnungskanal 5 dabei konisch ausgebildet, sodass der breitere Abschnitt des konischen Öffnungskanals 5 an der Ventilöffnung 4 angrenzt und der verjüngte Abschnitt des konischen Öffnungskanals 5 dazu entfernt weiter im Inneren des Ventils angeordnet ist. Der Ventilkörper 2 weist zudem einen, im Vergleich zu dem Öffnungskanal 5, größeren Kanal 16 des Ventilkörpers auf. Ein solcher größerer Kanal 16 schließt sich bzw. grenzt mit einer Schulter 17 an den Öffnungskanal 5 an.

Das Ventil 1 weist auch eine Fluidöffnung 3 auf. Mithilfe derer kann ein Fluid, welches sich in dem Ventil 1 befindet und/oder welches mittels eines Tubus 6 in das geöffnete Ventil 1 gebracht wurde, durch die Fluidöffnung 3 in ein angrenzendes, an die Fluidöffnung anschließendes, System übertragen bzw. transportiert werden. Ebenso ist es alternativ oder zusätzlich möglich, dass mithilfe der Fluidöffnung 3 ein Fluid, welches sich in einem angrenzende, an die Fluidöffnung anschließenden, System befindet, durch die Fluidöffnung 3 in das Ventil 1 und/oder durch das geöffnete Ventil 1 in einen Tubus 6 zu übertragen bzw. transportieren.

Im Ventilkörper 2 ist der Verschlusskörper 7 angeordnet. Er ist dabei im zweiten Abschnitt 11 radial komprimiert und im ersten Abschnitt zwischen dem Boden des Ventilkörpers 18 und der Schulter 17 in seiner Längserstreckung komprimiert.

In dem Längsschnitt der Figur 2 ist der Verschlusskörper im Schnitt der in der Figur 1 eingezeichnete Schnittebene AA zu erkennen, wobei er sich in der Figur 2 jedoch in einem deformierten Zustand und in der Figur 1 im undeformierten Zustand befindet. Durch die Anordnung des zweiten Abschnitts 11 im Öffnungskanal 5, wird der erste Abschnitt 11 so radial komprimiert, dass der zweite Fluidkanal 12 geschlossen ist.. Ein Schließen des Ventils wird dadurch erreicht, dass der Verschlusskörper 7 die Ventilöffnung 4 verschließt, und der zweite Abschnitt 11 in dem Öffnungskanal 5 angeordnet ist. Durch den Öffnungskanal 5 wird dabei der zweite Abschnitt 11 derart deformiert, dass der zweite Fluidkanal 12 verschlossen wird. Daher ist dieser in der Figur 2 nur als Strich und nicht als offener zweiter Fluidkanal 12 - wie in der Figur 1 - gezeigt. Um ein Schließen des zweiten Fluidkanals 12 zu bewirken und einen geschlossenes Ventil 1 zu erhalten, wurde bei diesem Ventil 1 der Öffnungskanal 5 im Verhältnis zum ersten Abschnitt des Ventilkörpers so eingerichtet, dass dieser eine radial wirkende Kraft auf den zweiten Abschnitt 11 ausübt. Dadurch wird der zweite Abschnitt 11 deformiert und komprimiert und somit der zweite Fluidkanal 12 verschlossen und verschlossen gehalten. Eine solche Kraft kann dadurch erzeugt werden, dass - wie in dem der Figur 2 gezeigten Ventil - der runde Innendurchmesser der Ventilöffnung 4 und eines Teilabschnitts des Öffnungskanals 5 kleiner ist, als die größte Achse des Querschnitts des zweiten Abschnitts 11. Aufgrund Komprimierung der der Längserstreckung des Verschlusskörpers 7 wird das Verschließen und besonders das geschlossen halten des zweiten Fluidkanals 12 begünstig.

Ein solches Ventil 1 mit einem derart geschlossenen zweiten Fluidkanal 12 kann dabei bei einem Druck bzw. einer Druckdifferenz des Innendrucks innerhalb des Verschlusskörpers 7 aufgrund einer darin befindlichen Flüssigkeit zu einem Außendruck des Ventils 1 von bis zu 0,21 MPa geschlossen verbleiben.

In dem ersten Abschnitt 9 des Verschlusskörpers 7 ist der erste Fluidkanal ausgebildet. Der erste Fluidkanal 10 weist dabei aufgrund der Geometrie des ersten Abschnitts 9 des Verschlusskörpers 7 eine solche Geometrie des Hohlraums des ersten Fluidkanals 10 auf, dass eine Sanduhr artige Struktur im Längsschnitt gegeben ist. Eine solche Struktur ist dadurch gekennzeichnet, dass eine verjüngte bzw. engere Stelle von einem darüber und darunter befindlichen breiteren Bereich des Fluidkanals umgeben ist. Eine solche geometrische Ausformung des ersten Fluidkanals 10 ist zu bevorzugen, da dadurch zum einen beim Entfernen des Tubus 6 aus der Ventilöffnung 4 der Verschlusskörper 7 sich wieder so elastisch zurückformt, dass ein Unterdruck beim Herausnehmen des Tubus 6 aus dem Ventil 1 erzeugt wird, sodass möglichst wenig bzw. kein Rest eines Fluids an einer Außenseite des Tubus 6 verbleibt.

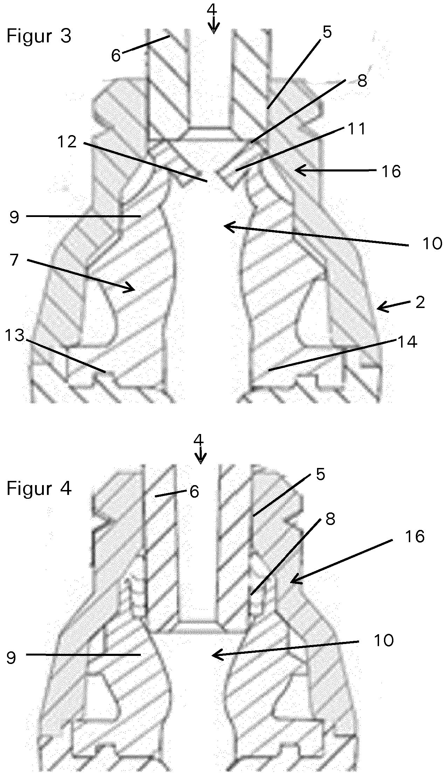

In der Figur 3 ist die Anordnung aus der Figur 2 wieder im Längsschnitt gezeigt, wobei der Tubus 6 durch die Ventilöffnung 4 in den Öffnungskanal 5 eingeführt wurde und dadurch das Ventil in einen zumindest teilweise geöffneten Zustand verbracht wurde. Dabei hat das Einführen des Tubus 6 eine Kraft auf die endständige Oberfläche 8 übertragen und wurde dadurch das Ventil 1 in einen zumindest teilweise offenen Zustand verbracht. In einem solchen Zustand ist der zweite Abschnitt 11 zumindest teilweise in den größeren Kanal 16 des Ventilkörpers 7 überführt worden und der zweite Fluidkanal 12 ermöglicht eine Verbindung zwischen Tubus und erstem Fluidkanal 10 bzw. weitergehend der Fluidöffnung 3. Eine solche Verbindung kann dabei genutzt werden, um Fluide zwischen Tubus 6 und Ventil 1 bzw. ein angrenzendes, an die Fluidöffnung 3 anschließendes, System auszutauschen oder Fluide aus dem Ventil 1 bzw. in das Ventil 1 zu transportieren. Dabei ist bei einem solchen, nicht vollständigen, Einführen des Tubus 6 der Verschlusskörper 7 derart deformiert, dass der zweite Fluidkanal 12 bereits dilatiert ist. Dabei ist der Verschlusskörper 7 so ausgebildet, dass während der Deformation der zweite Fluidkanal 12 nicht nur soweit geöffnet wird, wie es einem undeformierten Zustand des Verschlusskörpers 7 (vergleiche dazu Figur 1) entspräche, sondern ist der zweite Fluidkanal 12 über den undeformierten Zustand des Verschlusskörpers 7 hinaus dilatiert. Wie in der Figur 3 ersichtlich wurde der zweite Fluidkanal 12 dabei soweit dilatiert, sodass die Querschnittsfläche, also die Fläche im Querschnitt, die der zweite Fluidkanal zwecks Verbindung zwischen erstem Fluidkanal 10 und Tubus 6 freigibt, auf das 1,2-Fache der Querschnittsfläche des zweiten Fluidkanals 12 im undeformierten Zustand des Verschlusskörpers 7 vergrößert. Diese Dilatation wird allein durch den Tubus 6 und dessen Krafteinwirkung auf die endständige Oberfläche 8 erreicht und zwar außerhalb des zweiten Fluidkanals 12, und ohne weitere Hilfsmittel wie Sporne zum Aufweiten des Kanals innerhalb des Ventilkörpers 2 bzw. Verschlusskörpers 7 oder Ummantelungen um den Verschlusskörper 7.

Der Verschlusskörper 7 weist einen endständigen Teilabschnitt 12 auf, der an die Fluidöffnung 3 mit seiner Unterfläche angrenzt und auf einem Boden des Ventilkörpers aufliegt. Bei der Deformation zur Öffnung verbleibt diese Unterfläche ortsfest, wie ein Vergleich zwischen Figur 2, 3 und 4 zeigt. Somit stabilisiert ein solcher endständiger Teilabschnitt 12 den Verschlusskörper 7 bei der Deformation, wodurch die gewünschte Deformation und Dilatation besonders günstig verwirklich werden kann. Ebenso begünstigend wirken sich die Mittel 13, umlaufende Nut und Feder, in einem solchen endständigen Teilabschnitt 12 aus, die durch ihre Geometrie und Anordnung im Ventilkörper 2 und gegenüber der Fluidöffnung 3 ebenso das Öffnen und Schließen des Ventils begünstigend beeinflusst.

In der Figur 4 ist erneut die Anordnung aus der Figur 3 im Längsschnitt gezeigt, wobei der Tubus 6 durch die Ventilöffnung 4 in den Öffnungskanal 5 und den größeren Kanal 16 bis zum Anschlag an die Innenseite des ersten Fluidkanals 10 eingeführt wurde. Dabei erfolgte eine solche Dilatation des zweiten Fluidkanals 12, dass die endständige Oberfläche 8 im Grunde vollständig an der äußeren Mantelfläche des Tubus 6 anliegt und der Tubus in direkter Verbindung mit dem ersten Fluidkanal steht. Dabei stellt daher der zweite Fluidkanal 12 keine Verbindung zwischen Tubus 6 und erstem Fluidkanal 10 her, da der zweite Fluidkanal 12 soweit dilatiert wurde, dass er komplett durch den Tubus 6 verdrängt wurde. Somit lässt sich ein besonders effektiver Fluidaustausch bewirken. Dabei weist der erste Abschnitt 9 einen Teilabschnitt auf, der ein weiteres Einführen des Tubus 6 in den größeren Kanal 16 begrenzt und somit ein weiteres Einführen des Tubus 6 allein durch den Verschlusskörper 7 verhindert wird.

Ausgehend von diesem offenen Zustand wird beim Entfernen des Tubus 6 aus dem Öffnungskanal 5 und der Ventilöffnung 4, sodass z. B. keine Krafteinwirkung auf die endständige Oberfläche 8 vorhanden ist, der zweite Abschnitt 11 durch die teilweise Entspannung des Verschlusskörpers 7, insbesondere des ersten Abschnitts 9, so in den Öffnungskanal 5 zurückdrängt, dass der Ruhezustand des Ventils 1, wie in Figur 2 dargestellt, ohne Weiteres und sicher hergestellt wird.

## Patentansprüche

1. Ventil (1) aufweisend
einen Ventilkörper (2)
mit einer Ventilöffnung (4), die sich endständig an einem Öffnungskanal (5) des Ventilkörpers (2) befindet, wobei der Öffnungskanal (5) einen runden Innenquerschnitt aufweist und die Ventilöffnung und der Öffnungskanal zur Aufnahme eines Tubus (6) durch Einführen des Tubus (6) durch die Ventilöffnung (4) hindurch in den Öffnungskanal (5) eingerichtet sind, wobei das Ventil (1) einen elastisch deformierbaren Verschlusskörper (7) innerhalb des Ventilkörpers (2) aufweist,
wobei der Verschlusskörper (7) eine endständige Oberfläche (8) und in einem ersten Abschnitt (9) einen ersten Fluidkanal (10) aufweist, wobei der Verschlusskörper (7) so ausgebildet ist, dass er in einem undeformierten Zustand des Verschlusskörpers (7) in einem zweiten und endständigen Abschnitt (11) einen zweiten Fluidkanal (12) aufweist, der mit dem ersten Fluidkanal (10) verbunden ist, wobei der zweite Fluidkanal (12) an der endständigen Oberfläche (8) mündet und der zweite Abschnitt (11) im Querschnitt entlang zweier Hauptträgheitsachsen senkrecht zur Längserstreckung des Ventils (1) und/oder Verschlusskörpers (7) und/oder parallel zur Ebene der Ventilöffnung (4) und/oder endständigen Oberfläche (8) unterschiedliche Erstreckungen aufweist, wobei der runde Innendurchmesser des Öffnungskanals (5) kleiner ist als die größte Achse der Hauptträgheitsachsen des zweiten Abschnitts (11), wobei der zweite Fluidkanal im Querschnitt so ausgebildet ist, dass seine Form entlang zweier Hauptträgheitsachsen senkrecht zur Längserstreckung des Ventils und/oder Verschlusskörpers und/oder parallel zur Ebene der Ventilöffnung und/oder endständigen Oberfläche unterschiedliche Erstreckungen aufweist, wobei die Hauptträgheitsachse des zweiten Fluidkanals, in deren Richtung der zweite Fluidkanal seine kleinere Erstreckung aufweist, mit einem Winkel kleiner 20° zu der Hauptträgheitsachse des zweiten Abschnitts, in deren Richtung der zweite Abschnitt seine größere Erstreckung aufweist, angeordnet ist, wobei in einem Ruhezustand des Ventils (1), in dem das Ventil (1) durch den Verschlusskörper (7) verschlossen ist, der Verschlusskörper (7) mit seinem zweiten Abschnitt (11) in dem Öffnungskanal (5) des Ventilkörpers (2) so angeordnet ist, dass der zweite Abschnitt (11) so deformiert ist, dass der zweite Fluidkanal (12) verschlossen ist,
wobei das Ventil (1) so eingerichtet ist, dass durch Einführen des Tubus (6) durch die Ventilöffnung (4) in den Öffnungskanal (5) unter Ausübung einer Kraft mit dem Tubus (6) auf die endständige Oberfläche (8) das Ventil (1) in einen offenen Zustand verbracht wird, wobei der zweite Abschnitt (11) aus dem Öffnungskanal (5) in einen im Querschnitt größeren Kanal (16) des Ventilkörpers (2) überführt wird, und wobei in dem offenen Zustand der zweite Fluidkanal (12) eine Verbindung zwischen dem ersten Fluidkanal (10), insbesondere der Fluidöffnung (3), und dem Tubus (6) ermöglicht, und wobei das Ventil (1) so eingerichtet ist, dass ausgehend vom offenen Zustand beim Entfernen des Tubus (6) aus dem Öffnungskanal (5), und insbesondere der Ventilöffnung (4), der zweite Abschnitt (11) so in den Öffnungskanal (5) zurückdrängt wird, dass der Ruhezustand hergestellt wird,
wobei
das Ventil (1) so eingerichtet ist, dass unter Einführung des Tubus (6) durch die Ventilöffnung (4) in den Öffnungskanal (5) das Ventil (1) aus seiner Ruhelage in den offenen Zustand gebracht wird und dadurch der Verschlusskörper (7) so deformiert wird, dass
der zweite Fluidkanal (12) über den undeformierten Zustand des Verschlusskörpers (7) hinaus dilatiert wird,
**dadurch gekennzeichnet, dass** der Verschlusskörper (7) so ausgebildet ist, dass diese Dilatation unmittelbar und allein durch Krafteinwirkung auf die endständige Oberfläche (8) des Verschlusskörpers (7) mittels des Tubus (6) an der Oberfläche des Verschlusskörpers (7) außerhalb des zweiten Fluidkanals (12) zumindest soweit bewirkt wird, dass eine Vergrößerung der Querschnittsfläche des zweiten Fluidkanals (12) zumindest auf das 1,1 fache der Querschnittsfläche des zweiten Fluidkanals (12) im undeformierten Zustand des Verschlusskörpers (7) bewirkt wird.

2. Ventil (1) nach Anspruch 1 **dadurch gekennzeichnet, dass** der zweite Fluidkanal (12) gegenüber dem ersten Fluidkanal (10) verjüngt ist, und insbesondere zumindest eine Mehrzahl von Hohlraumabschnitten des Verschlusskörpers (7), insbesondere des ersten Fluidkanals (10), derart ausgebildet sind, dass zumindest ein gegenüber zumindest einem größerer Hohlraumabschnitt des Verschlusskörpers (7) verjüngter Hohlraumabschnitt oder mindestens ein zwischen zwei Hohlraumabschnitten angeordneter und gegenüber diesen Hohlraumabschnitten verjüngter Hohlraumabschnitt ausgebildet ist und/oder mindestens ein Hohlraumabschnitt Sanduhr artig, ausgebildet ist.

3. Ventil (1) nach einem der vorherigen Ansprüche **dadurch gekennzeichnet, dass** der zweite Abschnitt (11) im Ruhezustand des Ventils (1) eine Unterfläche ausbildet, an der der zweite Fluidkanal (12) mündet, die einen Winkel zu der Längserstreckung des Ventils (1) und/oder Verschlusskörpers (7) und/oder einer Ebene senkrecht zur Ebene der Ventilöffnung (4) und/oder der endständigen Oberfläche (8) von mehr als 75° und weniger als 115° aufweist.

4. Ventil nach einem der vorherigen Ansprüche **dadurch gekennzeichnet, dass** das Ventil (1) so ausgebildet ist, dass die Ventilöffnung (4) und/oder der Öffnungskanal (5) im Querschnitt senkrecht zur Längserstreckung des Ventils (1) und/oder Verschlusskörpers (7) und/oder parallel zur Ebene der Ventilöffnung (4) und/oder endständigen Oberfläche (8) zumindest eine erste Erstreckung zwischen geometrischen Mittelpunkt des Querschnitts und einem Punkt auf dem Außenumfang des Querschnitts aufweist, die kleiner ist als zumindest eine zweite Erstreckung zwischen geometrischen Mittelpunkt des Querschnitts des zweiten Abschnitts (11) senkrecht zur Längserstreckung des Ventils (1) und/oder Verschlusskörpers (7) und/oder parallel zur Ebene der Ventilöffnung (4) und/oder endständigen Oberfläche (8) und einem Punkt auf dem Außenumfang des Querschnitts des zweitens Abschnitts (11) im undeformierten Zustand des Verschlusskörpers (7), wobei die zumindest eine erste Strecke der Ventilöffnung (4) und/oder des Öffnungskanals (5) im geschlossen Zustand des Ventils (1) mit der zweiten, jedoch komprimierten Erstreckung zusammenfällt und/oder deckungsgleich ist.

5. Ventil (1) nach einem der vorherigen Ansprüche **dadurch gekennzeichnet, dass** der Verschlusskörper (7) so ausgebildet ist, dass zweite Fluidkanal (11) in einem undeformierten Zustand des Verschlusskörpers (7) im Querschnitt eine kleinste Achse mit einer Länge von mindestens 5% und/oder weniger als 25% der Querschnittserstreckung der Ventilöffnung (4) und/oder der Querschnittsachsenlänge des zweiten Fluidkanals (12) im geöffneten Zustand des Ventils (1) aufweist.

6. Ventil (1) nach einem der vorherigen Ansprüche **dadurch gekennzeichnet, dass** der erste Abschnitt (9) einen endständigen Teilabschnitt (14) aufweist, wobei der erste Fluidkanal (10), und insbesondere nicht der zweite Fluidkanal (12), an diesem mündet und/oder der endständigen Teilabschnitt (14) nicht an den zweiten Abschnitt (11) angrenzt, insbesondere ihm (11) gegenüberliegend angeordnet ist und/oder durch einen Zwischenteilabschnitt des ersten Abschnitts (9) vom zweiten Abschnitt (11) getrennt ist, und insbesondere der endständigen Teilabschnitt (14) an einer endständigen Fluidöffnung (3) oder einem endständigen Fluidkanal des Ventils (1) zumindest teilweise anliegt, wobei
der endständige Teilabschnitt (14) die größte Breite, die größte Querschnittsfläche und/oder den größten Umfang des Verschlusskörpers (7) aufweist und/oder wobei dieser Teilabschnitt (14) mindestens ein Rastmittel und/oder eine Nut (13) aufweist, und/der wobei das Ventil (1) so ausgebildet ist, dass der Teilabschnitt (14) im Ruhezustand, im geöffneten Zustand des Ventils (1) und in allen Zuständen beim Öffnen des Ventils (1) aus dem Ruhezustand heraus und in allen Zuständen beim Überführen vom geöffneten Zustand in den Ruhezustand der endständige Teilabschnitt (14) auf einem Boden (18) des Ventilgehäuses aufliegt, wobei sich insbesondere vom Boden (18) aus der endständige Fluidkanal zur endständigen Fluidöffnung (3) erstreckt oder die endständigen Fluidöffnung (3) im Boden (18) angeordnet ist und/oder der Boden (18) auch eine Begrenzung des breiten Kanals (16) des Ventilkörpers (4) darstellt und/oder wobei
der endständige Teilabschnitt (14) eine Bodenfläche des Verschlusskörpers (7) definiert, die im Ruhezustand des Ventils (1) in einer ersten Lage oder Ebene angeordnete ist und im geöffneten Zustand des Ventils (1) in einer zweiten Ebene oder Lage angeordnet ist, wobei die erste Lage oder Ebene und die zweite Lage oder Ebene um weniger als 5% der Längserstreckung des Verschlusskörpers (7) in Längserstreckung des Verschlusskörpers (7) verschoben sind, wobei die erste und die zweite Ebene insbesondere parallel zueinander und/oder senkrecht zur Längserstreckung des Ventils (1) und/oder Verschlusskörpers (7) und/oder parallel zur Ebene der Ventilöffnung (4) und/oder endständigen Oberfläche (8) liegen.

7. Ventil (1) nach einem der vorherigen Ansprüche **dadurch gekennzeichnet, dass** das Ventil (1) so eingerichtet ist, dass
im geöffneten Zustand des Ventils (1) durch Einführung des Tubus (6) durch die Ventilöffnung (4), insbesondere nur bis maximal zum Ende des Öffnungskanals (5) und/oder bis zum Beginn des breiteren Kanals (16), eine Strecke auf dem zweiten Abschnitt, die sich zwischen einem Punkt auf dem Außenumfang des zweiten Fluidkanals (12) und einem Punkt auf dem Außenumfang des zweiten Abschnitts (11) erstreckt, die einen Schnittwinkel (γ) zu einer Ebene, die parallel zur Ebene der Ventilöffnung (4) im Ventilkörper (2) und/oder senkrecht zur Längserstreckung des Ventils (1) und/oder Verschlusskörpers (7) und/oder parallel zur endständigen Oberfläche (8), aufweist, der größer als 5° und/oder kleiner als 355° ist. und/oder dass

8. Ventil (1) nach einem der vorherigen Ansprüche **dadurch gekennzeichnet, dass** das Ventil (1) so ausgebildet ist, dass
der Verschlusskörper (7) bei Einführung des Tubus (6) durch die Ventilöffnung (4) und den Öffnungskanal (5) bis in den größeren Kanal (16) des Ventilkörpers (2) so verformt wird, dass die endständige Oberfläche (8) des Verschlusskörpers (7) an der äußeren Mantelfläche des Tubus (6) zumindest teilweise anliegt.

9. Ventil (1) nach einem der vorherigen Ansprüche, **dadurch gekennzeichnet, dass** auf und/oder an dem Verschlusskörper (7) eine Mehrzahl an Erhebungen, insbesondere Stege, angeordnet ist, die so eingerichtet sind, dass sie im Ruhezustand des Ventils (1) am Ventilkörper (2) anliegen und in den Bereich zwischen ihnen als Abstandshalter zwischen Ventilkörper (2) und Verschlusskörper (7) dienen.

10. Ventil (1) nach einem der vorherigen Ansprüche, wobei der Verschlusskörper (7) und/oder der Ventilkörper (2) so ausgebildet ist, dass im Ruhezustand des Ventils (1) nicht die gesamte Außenumfangsfläche des zweiten Abschnitts (11) an der Innenseite des Ventilkörpers (2) anliegt.

11. Ventil (1) nach einem der vorherigen Ansprüche, wobei der Verschlusskörper (7) so eingerichtet ist, dass der Verschlusskörper (7) im Ruhezustand des Ventils (1) nur teilweise an der Innenwand des Ventilkörpers (2) und/oder dem breiteren Kanal (16) anliegt.

12. Verfahren zum Öffnen und Schließen eines Ventils (1) nach einem der voranstehenden Ansprüchen mit einem, insbesondere nadellosen, Tubus (6), wobei zum Schließen des Ventils der Verschlusskörper (7) des Ventils oder der zweite Abschnitt (11) des Verschlusskörpers (7) in den runden Öffnungskanal (5) des Ventilkörpers (2) des Ventils verbracht und/oder in den runden Öffnungskanal (5) des Ventilkörpers (2) des Ventils angeordnet wird, so dass der Verschlusskörper (7) die Öffnung (4) des Ventilkörpers (2) verschließt, wobei die Öffnung insbesondere eine am Ventil außen liegende und/oder endständige ist, wobei der Öffnungskanal (5) den Verschlusskörper (7) oder den Abschnitt (11), insbesondere radial, komprimiert, so dass der zweite Fluidkanal (12) geschlossen wird und
wobei zum Öffnen der Verschlusskörper (7) oder der zweite Abschnitt (11) durch Drücken mit dem Tubus (6) auf den Verschlusskörper (7) oder den zweiten Abschnitt (11), jedoch außerhalb des zweiten Fluidkanals (12), ohne Ausüben von radial weitenden Kräften im zweiten Fluidkanal (12) in einen gegenüber dem Öffnungskanal (5) weiteren und/oder breiteren Kanal (16) des Ventils, insbesondere des Ventilkörpers (2), verbracht wird, der den Verschlusskörper (7) oder den zweiten Abschnitt (11) zumindest um 10% weniger, insbesondere nicht, komprimiert, wobei anschließend durch weiteres Einführen des Tubus (6) in das Ventil unter Drücken mit dem Tubus (6) auf die Verschlusskörperoberfläche (8) den Fluidkanal (12) weitende Kräfte eingebracht werden und der Tubus (6) in den Fluidkanal (12) eingeführt wird.

13. Verfahren zum Öffnen und Schließen eines Fluidkanals (12) eines Ventils nach Anspruch 12, wobei bei Einführen des Tubus (6) der Fluidkanal (12) derart radial geweitet wird, dass der Tubus (6) so in das Ventil, insbesondere den Fluidkanal (12), eingeführt wird, dass mindestens 50% des Querschnitts des Innenkanals des Tubus (6) innerhalb des Ventils, insbesondere des Fluidkanals (12) als durch das Ventil, insbesondere gradlinig, durchgehender Kanal, freigegeben wird.

14. Verfahren nach einem der beiden voranstehenden Ansprüche, wobei beim Entfernen des Tubus (6) durch teilweises Entspannen des Verschlusskörpers (7) ein Unterdruck im ersten Fluidkanal und/oder breiteren Kanal erzeugt wird.

## Claims

1. A valve (1) comprising
a valve body (2)
with a valve opening (4) which is located at the end of an opening channel (5) of the valve body (2), wherein the opening channel (5) has a round internal cross-section and the valve opening and the opening channel are designed to receive a tube (6) by inserting the tube (6) through the valve opening (4) into the opening channel (5),
wherein the valve (1) has an elastically deformable closure body (7) within the valve body (2),
wherein the closure body (7) has an end surface (8) and a first fluid channel (10) in a first section (9), wherein the closure body (7) is designed such that, in an undeformed state of the closure body (7), it has a second fluid channel (12) in a second and terminal section (11), which is connected to the first fluid channel (10), wherein the second fluid channel (12) opens at the end surface (8) and the second section (11) has different extents in cross-section along two main axes of inertia perpendicular to the longitudinal extent of the valve (1) and/or closure body (7) and/or parallel to the plane of the valve opening (4) and/or end surface (8),
wherein the round inner diameter of the opening channel (5) is smaller than the largest axis of the main axes of inertia of the second section (11),
wherein the cross-section of the second fluid channel is such that its shape has different extents along two main axes of inertia perpendicular to the longitudinal extent of the valve and/or closure body and/or parallel to the plane of the valve opening and/or end surface,
wherein the main axis of inertia of the second fluid channel, in the direction of which the second fluid channel has its smaller extension, is arranged at an angle of less than 20° to the main axis of inertia of the second section, in the direction of which the second section has its larger extension,
wherein in an idle state of the valve (1), in which the valve (1) is closed by the closure body (7), the closure body (7) is arranged with its second section (11) in the opening channel (5) of the valve body (2), so that the second section (11) is deformed in such a way that the second fluid channel (12) is closed,
wherein the valve (1) is arranged such that the valve (1) is brought into an open state by inserting the tube (6) through the valve opening (4) into the opening channel (5) while exerting a force with the tube (6) on the end surface (8),
wherein the second section (11) is transferred from the opening channel (5) into a channel (16) of the valve body (2) with a larger cross-section, and
wherein in the open state the second fluid channel (12) enables a connection between the first fluid channel (10), in particular the fluid opening (3), and the tube (6), and
wherein the valve (1) is arranged such that, starting from the open state when the tube (6) is removed from the opening channel (5), and in particular the valve opening (4), the second section (11) is pushed back into the opening channel (5) such that the rest state is established,
wherein the valve (1) is arranged in such a way that when the tube (6) is inserted through the valve opening (4) into the opening channel (5), the valve (1) is brought from its rest position into the open state, thereby deforming the closure body (7), so that the second fluid channel (12) is dilated beyond the undeformed state of the closure body (7),
**characterised in that** the closure body (7) is designed such that this dilation is brought about directly and solely by the action of force on the end surface (8) of the closure body (7) by means of the tube (6) on the surface of the closure body (7) outside the second fluid channel (12) at least to such an extent that an increase in the cross-sectional area of the second fluid channel (12) to at least 1.1 times the cross-sectional area of the second fluid channel (12) in the undeformed state of the closure body (7) is brought about.

2. A valve (1) according to claim 1, **characterised in that** the second fluid channel (12) is tapered relative to the first fluid channel (10), and in particular at least a plurality of cavity sections of the closure body (7), in particular of the first fluid channel (10), are formed in such a way, so that at least one cavity section tapered relative to at least one larger cavity section of the closure body (7) or at least one cavity section arranged between two cavity sections and tapered relative to these cavity sections is formed and/or at least one cavity section is formed in the manner of an hourglass.

3. A valve (1) according to one of the preceding claims, **characterised in that** the second section (11), in the rest state of the valve (1), forms a lower surface at which the second fluid channel (12) opens, which has an angle to the longitudinal extent of the valve (1) and/or closure body (7) and/or a plane perpendicular to the plane of the valve opening (4) and/or the end surface (8) of more than 75° and less than 115°.

4. A valve according to one of the preceding claims, **characterised in that** the valve (1) is designed such that the valve opening (4) and/or the opening channel (5) has in cross-section perpendicular to the longitudinal extent of the valve (1) and/or closure body (7) and/or parallel to the plane of the valve opening (14) and/or end surface (8) at least a first extent between the geometric centre of the cross-section and a point on the outer circumference of the cross-section, which is smaller than at least a second extension between the geometric centre of the cross-section of the second section (11) perpendicular to the longitudinal extension of the valve (1) and/or closure body (7) and/or parallel to the plane of the valve opening (4) and/or end surface (8) and a point on the outer circumference of the cross-section of the second section (11) in the undeformed state of the closure body (7),
wherein the at least one first section of the valve opening (4) and/or of the opening channel (5) in the closed state of the valve (1) coincides and/or is congruent with the second, but compressed extension.

5. A valve (1) according to one of the preceding claims, **characterised in that** the closure body (7) is designed such that the second fluid channel (11) in an undeformed state of the closure body (7) has a smallest axis in cross-section with a length of at least 5% and/or less than 25% of the cross-sectional extent of the valve opening (4) and/or the cross-sectional axis length of the second fluid channel (12) in the open state of the valve (1).

6. A valve (1) according to one of the preceding claims, **characterised in that** the first section (9) has an end partial section (14), wherein the first fluid channel (10), and in particular not the second fluid channel (12), opens at this and/or the end partial section (14) is not adjacent to the second section (11), in particular is arranged opposite it (11) and/or is separated from the second section (11) by an intermediate partial section of the first section (9), and in particular the end partial section (14) bears at least partially against a terminal fluid opening (3) or a terminal fluid channel of the valve (1),
wherein the end partial section (14) has the largest width, the largest cross-sectional area and/or the largest circumference of the closure body (7) and/or wherein this partial section (14) has at least one latching means and/or a groove (13), and/or
wherein the valve (1) is designed in such a way that the end portion (14) rests on a base (18) of the valve housing in the idle state, in the open state of the valve (1) and in all states when the valve (1) is opened from the idle state and in all states when the valve (1) is transferred from the open state to the idle state,
wherein, in particular, the terminal fluid channel extends from the base (18) to the terminal fluid opening (3) or the terminal fluid opening (3) is arranged in the base (18) and/or the base (18) also forms a boundary of the wide channel (16) of the valve body (4) and/or
wherein the end partial section (14) defines a bottom surface of the closure body (7), which is arranged in a first position or plane when the valve (1) is in the closed state and is arranged in a second plane or position when the valve (1) is in the open state,
wherein the first layer or plane and the second layer or plane are displaced by less than 5% of the longitudinal extent of the closure body (7) in the longitudinal extent of the closure body (7),
wherein the first and second planes are in particular parallel to each other and/or perpendicular to the longitudinal extension of the valve (1) and/or closure body (7) and/or parallel to the plane of the valve opening (4) and/or end surface (8).

7. A valve (1) according to one of the previous claims, **characterised in that** the valve (1) is set up in such a way that, in the open state of the valve (1), by inserting the tube (6) through the valve opening (4), in particular only up to a maximum of the end of the opening channel (5) and/or up to the beginning of the wider channel (16), a distance on the second section, which extends between a point on the outer circumference of the second fluid channel (12) and a point on the outer circumference of the second section (11), which has an angle of intersection (γ) with a plane which is parallel to the plane of the valve opening (4) in the valve body (2) and/or perpendicular to the longitudinal extension of the valve (1) and/or closure body (7) and/or parallel to the end surface (8), which is greater than 5° and/or less than 355°.

8. A valve (1) according to one of the preceding claims, **characterised in that** the valve (1) is designed such that the closure body (7) is deformed when the tube (6) is inserted through the valve opening (4) and the opening channel (5) into the larger channel (16) of the valve body (2) such that the end surface (8) of the closure body (7) rests at least partially against the outer circumferential surface of the tube (6).

9. A valve (1) according to one of the preceding claims, **characterised in that** a plurality of elevations, in particular webs, are arranged on and/or on the closure body (7), which are arranged in such a way that they rest against the valve body (2) when the valve (1) is at rest and serve as spacers between the valve body (2) and the closure body (7) in the region between them.

10. A valve (1) according to one of the preceding claims, wherein the closure body (7) and/or the valve body (2) is designed such that in the rest state of the valve (1) not the entire outer circumferential surface of the second section (11) is in contact with the inside of the valve body (2).

11. A valve (1) according to one of the preceding claims, wherein the closure body (7) is arranged such that the closure body (7) only partially abuts the inner wall of the valve body (2) and/or the wider channel (16) when the valve (1) is at rest.

12. A method for opening and closing a valve (1) according to one of the preceding claims with a, in particular needleless, tube (6), wherein, for closing the valve, the closure body (7) of the valve or the second section (11) of the closure body (7) is brought into the round opening channel (5) of the valve body (2) of the valve and/or is arranged in the round opening channel (5) of the valve body (2) of the valve, so that the closure body (7) closes the opening (4) of the valve body (2),
wherein the opening is in particular an external and/or terminal opening on the valve, wherein the opening channel (5) compresses the closure body (7) or the section (11), in particular radially, so that the second fluid channel (12) is closed, and
wherein, for opening, the closure body (7) or the second section (11) is moved by pressing with the tube (6) on the closure body (7) or the second section (11), but outside the second fluid channel (12), without exerting radially expanding forces in the second fluid channel (12) into a wider and/or wider channel (16) of the valve, in particular of the valve body (2), relative to the opening channel (5), which compresses the closure body (7) or the second section (11) at least 10% less, in particular not at all, wherein subsequently, by further insertion of the tube (6) into the valve, forces are introduced with the tube (6) onto the closure body surface (8) to widen the fluid channel (12) and the tube (6) is inserted into the fluid channel (12).

13. A method for opening and closing a fluid channel (12) of a valve according to claim 12, wherein, when the tube (6) is introduced, the fluid channel (12) is widened radially in such a way that the tube (6) is introduced into the valve, in particular the fluid channel (12), in such a way that at least 50% of the cross-section of the inner channel of the tube (6) is released within the valve, in particular the fluid channel (12), as a channel passing through the valve, in particular in a straight line.

14. A Method according to one of the two preceding claims, wherein, when removing the tube (6), a negative pressure is generated in the first fluid channel and/or wider channel by partial relaxation of the closure body (7).

## Revendications

1. Soupape (1) comprenant
un corps de soupape (2)
avec une ouverture de soupape (4) située à l'extrémité d'un canal d'ouverture (5) du corps de soupape (2), dans lequel le canal d'ouverture (5) a une section transversale interne ronde et l'ouverture de soupape et le canal d'ouverture sont conçus pour recevoir un tube (6) en insérant le tube (6) à travers l'ouverture de soupape (4) dans le canal d'ouverture (5),
dans lequel la soupape (1) possède un corps de fermeture élastiquement déformable (7) à l'intérieur du corps de soupape (2),
dans lequel le corps de fermeture (7) a une surface d'extrémité (8) et un premier canal de fluide (10) dans une première section (9), dans lequel le corps de fermeture (7) est conçu de telle sorte que, dans un état non déformé du corps de fermeture (7), il a un second canal de fluide (12) dans une seconde section terminale (11), qui est reliée au premier canal de fluide (10),
dans lequel le second canal de fluide (12) s'ouvre à la surface d'extrémité (8) et la seconde section (11) a des étendues différentes en section transversale le long de deux axes principaux d'inertie perpendiculaires à l'étendue longitudinale de la soupape (1) et/ou du corps de fermeture (7) et/ou parallèles au plan de l'ouverture de soupape (4) et/ou de la surface d'extrémité (8),
dans lequel le diamètre intérieur rond du canal d'ouverture (5) est inférieur au plus grand axe des axes principaux d'inertie de la seconde section (11),
dans lequel la section transversale du second canal de fluide est telle que sa forme a des extensions différentes le long de deux axes principaux d'inertie perpendiculaires à l'étendue longitudinale de la soupape et/ou du corps de fermeture et/ou parallèles au plan de l'ouverture de la soupape et/ou de la surface d'extrémité,
dans lequel l'axe principal d'inertie du second canal de fluide, dans la direction où le second canal de fluide a sa plus petite extension, est disposé à un angle inférieur à 20° par rapport à l'axe principal d'inertie de la seconde section, dans la direction où la seconde section a sa plus grande extension,
dans lequel, dans un état de repos de la soupape (1), dans lequel la soupape (1) est fermée par le corps de fermeture (7), le corps de fermeture (7) est disposé avec sa seconde section (11) dans le canal d'ouverture (5) du corps de soupape (2), de sorte que la seconde section (11) est déformée de manière à ce que le second canal de fluide (12) soit fermé,
dans lequel la soupape (1) est disposée de manière à ce que la soupape (1) soit ouverte en insérant le tube (6) à travers l'ouverture de soupape (4) dans le canal d'ouverture (5) tout en exerçant une force avec le tube (6) sur la surface d'extrémité (8),
dans lequel la seconde section (11) est transférée du canal d'ouverture (5) dans un canal (16) du corps de soupape (2) avec une section transversale plus grande, et
dans lequel, à l'état ouvert, le second canal de fluide (12) permet une connexion entre le premier canal de fluide (10), en particulier l'ouverture de fluide (3), et le tube (6), et
dans lequel la soupape (1) est agencée de manière à ce que, à partir de l'état ouvert lorsque le tube (6) est retiré du canal d'ouverture (5), et en particulier de l'ouverture de soupape (4), la seconde section (11) soit repoussée dans le canal d'ouverture (5) de manière à ce que l'état de repos s'établisse,
dans lequel la soupape (1) est disposée de telle sorte que lorsque le tube (6) est inséré à travers l'ouverture de soupape (4) dans le canal d'ouverture (5), la soupape (1) est amenée de sa position de repos à l'état ouvert, déformant ainsi le corps de fermeture (7), de sorte que le second canal de fluide (12) est dilaté au-delà de l'état non déformé du corps de fermeture (7),
**caractérisé en ce que** le corps de fermeture (7) est conçu de manière à ce que cette dilatation soit provoquée directement et uniquement par l'action d'une force sur la surface d'extrémité (8) du corps de fermeture (7) au moyen du tube (6) sur la surface du corps de fermeture (7) à l'extérieur du second canal de fluide (12), au moins dans une mesure telle qu'une augmentation de la section transversale du second canal de fluide (12) à au moins 1,1 fois la section transversale du second canal de fluide (12) à l'état non déformé du corps de fermeture (7) est provoquée.

2. Soupape (1) selon la revendication 1, **caractérisée en ce que** le second canal de fluide (12) est conique par rapport au premier canal de fluide (10), et en particulier au moins une pluralité de sections de cavité du corps de fermeture (7), en particulier du premier canal de fluide (10), sont formées de telle manière, de sorte qu'au moins une section de cavité conique par rapport à au moins une section de cavité plus grande du corps de fermeture (7) ou au moins une section de cavité disposée entre deux sections de cavité et conique par rapport à ces sections de cavité est formée et/ou au moins une section de cavité est formée à la manière d'un sablier.

3. Soupape (1) selon l'une des revendications précédentes, **caractérisée en ce que** la seconde section (11), à l'état de repos de la soupape (1), forme une surface inférieure sur laquelle s'ouvre le second canal de fluide (1 2), qui présente un angle par rapport à l'étendue longitudinale de la soupape (1) et/ou du corps de fermeture (7) et/ou un plan perpendiculaire au plan de l'ouverture de soupape (4) et/ou la surface d'extrémité (8) de plus de 75° et de moins de 115°.

4. Soupape selon l'une des revendications précédentes, **caractérisé en ce que** la soupape (1) est conçu de telle sorte que l'ouverture de soupape (4) et/ou le canal d'ouverture (5) présente en section perpendiculaire à l'extension longitudinale de la soupape (1) et/ou du corps de fermeture (7) et/ou parallèle au plan de l'ouverture de soupape (14) et/ou de la surface d'extrémité (8) au moins une première extension entre le centre géométrique de la section transversale et un point de la circonférence extérieure de la section transversale, qui est plus petite qu'au moins une seconde extension entre le centre géométrique de la section de la seconde partie (11) perpendiculaire à l'extension longitudinale de la soupape (1) et/ou du corps de fermeture (7) et/ou parallèle au plan de l'ouverture de soupape (4) et/ou de la surface d'extrémité (8) et un point sur la circonférence extérieure de la section de la seconde partie (11) à l'état non déformé du corps de fermeture (7), dans lequel la première section au moins de l'ouverture de soupape (4) et/ou du canal d'ouverture (5) à l'état fermé de la soupape (1) coïncide et/ou est congruente avec la seconde extension, mais comprimée.

5. Soupape (1) selon l'une des revendications précédentes, **caractérisée en ce que** le corps de fermeture (7) est conçu de telle sorte que le second canal de fluide (11) à l'état non déformé du corps de fermeture (7) présente un plus petit axe en section avec une longueur d'au moins 5% et/ou moins de 25% de l'étendue en section de l'ouverture de soupape (4) et/ou de la longueur de l'axe en section du second canal de fluide (12) à l'état ouvert de la soupape (1).

6. Soupape (1) selon l'une des revendications précédentes, **caractérisée en ce que** la première section (9) présente une section partielle d'extrémité (14), dans laquelle le premier canal de fluide (10), et en particulier pas le second canal de fluide (12), débouche et/ou la section partielle d'extrémité (14) n'est pas adjacente à la seconde section (11), en particulier est disposée en face de celle-ci (11) et/ou est séparée de la seconde section (11) par une section partielle intermédiaire de la première section (9), et en particulier la section partielle d'extrémité (14) s'appuie au moins partiellement contre une ouverture de fluide terminale (3) ou un canal de fluide terminal de la soupape (1),
dans laquelle la section partielle d'extrémité (14) présente la plus grande largeur, la plus grande surface de section transversale et/ou la plus grande circonférence du corps de fermeture (7) et/ou dans laquelle cette section partielle (14) comporte au moins un moyen de verrouillage et/ou une rainure (13), et/ou
dans lequel la soupape (1) est conçue de telle sorte que la partie terminale (14) repose sur une base (18) du boîtier de la soupape à l'état de repos, à l'état ouvert de la soupape (1) et dans tous les états lorsque la soupape (1) est ouverte à partir de l'état de repos et dans tous les états lorsque la soupape (1) est transférée de l'état ouvert à l'état de repos,
dans lequel, en particulier, le canal de fluide terminal s'étend de la base (18) à l'ouverture de fluide terminal (3) ou l'ouverture de fluide terminal (3) est disposée dans la base (18) et/ou la base (18) forme également une limite du canal large (16) du corps de soupape (4) et/ou
dans lequel la section partielle d'extrémité (14) définit une surface inférieure du corps de fermeture (7), qui est disposée dans une première position ou un premier plan lorsque la soupape (1) est à l'état fermé et est disposée dans un second plan ou une seconde position lorsque la soupape (1) est à l'état ouvert,
dans lequel la première couche ou le premier plan et la seconde couche ou le second plan sont déplacés de moins de 5 % de l'étendue longitudinale du corps de fermeture (7) dans l'étendue longitudinale du corps de fermeture (7),
dans lequel le premier et le second plan sont en particulier parallèles l'un à l'autre et/ou perpendiculaires à l'extension longitudinale de la soupape (1) et/ou du corps de fermeture (7) et/ou parallèles au plan de l'ouverture de soupape (4) et/ou de la surface d'extrémité (8).

7. Soupape (1) selon l'une des revendications précédentes, **caractérisée en ce que** la soupape (1) est configurée de telle sorte qu'à l'état ouvert de la soupape (1), en insérant le tube (6) à travers l'ouverture de soupape (4), en particulier seulement jusqu'à un maximum de l'extrémité du canal d'ouverture (5) et/ou jusqu'au début du canal plus large (16), une distance sur la seconde section, qui s'étend entre un point de la circonférence extérieure du second canal de fluide (12) et un point de la circonférence extérieure de la seconde section (11), qui présente un angle d'intersection (γ) avec un plan parallèle au plan de l'ouverture de soupape (4) dans le corps de soupape (2) et/ou perpendiculaire à l'extension longitudinale de la soupape (1) et/ou du corps de fermeture (7) et/ou parallèle à la surface d'extrémité (8), qui est supérieur à 5° et/ou inférieur à 355°.

8. Soupape (1) selon l'une des revendications précédentes, **caractérisée en ce que** la soupape (1) est conçue de manière à ce que le corps de fermeture (7) soit déformé lorsque le tube (6) est inséré à travers l'ouverture de soupape (4) et le canal d'ouverture (5) dans le canal plus large (16) du corps de soupape (2) de manière à ce que la surface d'extrémité (8) du corps de fermeture (7) repose au moins partiellement sur la surface circonférentielle externe du tube (6).

9. Soupape (1) selon l'une des revendications précédentes, **caractérisée en ce qu'**une pluralité d'élévations, en particulier des bandes, sont disposées sur et/ou sur le corps de fermeture (7), qui sont disposées de telle sorte qu'elles s'appuient contre le corps de soupape (2) lorsque la soupape (1) est au repos et servent d'espacement entre le corps de soupape (2) et le corps de fermeture (7) dans la zone située entre eux.

10. Soupape (1) selon l'une des revendications précédentes, dans laquelle le corps de fermeture (7) et/ou le corps de soupape (2) est conçu de telle sorte qu'à l'état de repos de la soupape (1), la totalité de la surface circonférentielle extérieure de la seconde section (11) n'est pas en contact avec l'intérieur du corps de soupape (2).

11. Soupape (1) selon l'une des revendications précédentes, dans laquelle le corps de fermeture (7) est disposé de manière à ce que le corps de fermeture (7) n'entre que partiellement en contact avec la paroi intérieure du corps de soupape (2) et/ou le canal plus large (16) lorsque la soupape (1) est au repos.

12. Procédé d'ouverture et de fermeture d'une soupape (1) selon l'une des revendications précédentes avec un tube (6), en particulier sans aiguille, dans lequel, pour fermer la soupape, le corps de fermeture (7) de la soupape ou la seconde section (11) du corps de fermeture (7) est amené dans le canal d'ouverture rond (5) du corps de soupape (2) de la soupape et/ou est disposé dans le canal d'ouverture rond (5) du corps de soupape (2) de la soupape, de sorte que le corps de fermeture (7) ferme l'ouverture (4) du corps de soupape (2),
dans lequel l'ouverture est en particulier une ouverture externe et/ou terminale sur la soupape, dans lequel le canal d'ouverture (5) comprime le corps de fermeture (7) ou la section (11), en particulier radialement, de sorte que le second canal de fluide (12) est fermé, et
dans lequel, pour l'ouverture, le corps de fermeture (7) ou la seconde section (11) est déplacé en appuyant avec le tube (6) sur le corps de fermeture (7) ou la seconde section (11), mais en dehors du second canal de fluide (12), sans exercer de forces d'expansion radiale dans le second canal de fluide (12) dans un canal plus large et/ou plus large (16) de la soupape, en particulier du corps de soupape (2), par rapport au canal d'ouverture (5), qui comprime le corps de fermeture (7) ou la seconde section (11) au moins 10 % de moins, en particulier pas du tout, dans lequel ensuite, par une nouvelle insertion du tube (6) dans la soupape, des forces sont introduites avec le tube (6) sur la surface du corps de fermeture (8) pour élargir le canal de fluide (12) et le tube (6) est inséré dans le canal de fluide (12).

13. Procédé d'ouverture et de fermeture d'un canal de fluide (12) d'une soupape selon la revendication 12, dans lequel, lors de l'introduction du tube (6), le canal de fluide (12) est élargi radialement de telle sorte que le tube (6) est introduit dans la soupape, en particulier dans le canal de fluide (12), de telle sorte qu'au moins 50% de la section transversale du canal intérieur du tube (6) est libérée à l'intérieur de la soupape, en particulier dans le canal de fluide (12), comme un canal traversant la soupape, en particulier en ligne droite.

14. Procédé selon l'une des deux revendications précédentes, dans lequel, lors du retrait du tube (6), une pression négative est générée dans le premier canal de fluide et/ou le canal plus large par relaxation partielle du corps de fermeture (7).
